# EUROPEAN PATENT SPECIFICATION

(11) **EP 4 547 871 B1**
(45) Date of publication and mention of the grant of the patent: **14.01.2026**
(21) Application number: 23785986.3
(22) Date of filing: 29.09.2023
(51) Int. Cl.: C12Q 1/6886, C12Q 1/6888, C12Q 1/70

(54) **A METHOD, USES, SYSTEM AND KIT FOR DETECTING A HUMAN PAPILLOMAVIRUS GENOTYPE ASSOCIATED WITH DEVELOPMENT OF CERVICAL, ORAL OR ANOGENITAL CANCER IN A SUBJECT AND FOR DETERMINING THE GENOTYPE OR GENOTYPES THEREOF**
VERFAHREN, VERWENDUNGEN, SYSTEM UND KIT ZUM NACHWEIS EINES MENSCHLICHEN PAPILLOMAVIRUS-GENOTYPS
MÉTHODE, UTILISATIONS, SYSTÈME ET KIT POUR DÉTECTER UN GÉNOTYPE DU PAPILLOMAVIRUS HUMAIN ASSOCIÉ AU DÉVELOPPEMENT DU CANCER

(30) Priority: 30.09.2022 EP 22382913
(43) Date of publication of application: 07.05.2025
(73) Proprietor: Vitro, S.A., 41020 Sevilla (ES)
(72) Inventor: OLMO SEVILLA, Asunción, 18003 Granada (ES); PÉREZ GUERRERO, Laura, 18007 Granada (ES); DURÁN LOBATO, Marco Jesús, 18007 Granada (ES); FERNÁNDEZ DELGADO, Fátima, 18563 Granada (ES); CARRERO LÉRIDA, Juana, 18100 Armilla, Granada (ES); ÁLVAREZ FERNÁNDEZ, María Stela, 18690 Almuñecar, Granada (ES); JIMÉNEZ FERNÁNDEZ, Ángel Francisco, 41012 Sevilla (ES); DE LAS HERAS DE LÓZAR, Francisco, 41011 Sevilla (ES); JIMÉNEZ GONZÁLEZ, Daniel, 41013 Sevilla (ES); CASCALES BARRIO, Jesús, 41018 Sevilla (ES)
(74) Representative: Manuel Illescas y Asociados, S.L.U.
(86) International application number: PCT/EP2023/077006
(87) International publication number: WO 2024/068901

(56) References cited:
- US-A1- 2007 207 456
- US-A1- 2020 199 678

## Description

### Field of the invention

The present invention relates to the field of diagnostics, in particular to the field of detection of infection in a subject and determination of the specific genotype responsible for infection.

### Background to the invention

It is the problem of the present invention to provide a method, uses of system and kit which allows detection of a human papillomavirus (HPV) genotype associated with development of cervical, oral or anogenital cancer in a subject, as well as determination of said genotype or genotypes of human papillomavirus infecting said subject when said human papillomavirus genotype associated with development of cervical, oral or anogenital cancer is detected therein, wherein the quantities of reagents and materials used are minimized. In addition, it is the problem of the present invention to provide a method, uses of wees system and kit for these purposes which is accurate, precise and fully automated and may be based on a sample taken by said subject from themselves, without need for intervention by a doctor or other medical personnel, thus being fully automated from subject to result. Moreover, it is the problem of the present invention to provide a method, uses of system and kit for these purposes which is cheaper and faster.

Document US2020/199678 discloses a method for detection of HPV types 16, 18, 31, 33, 35, 39, 45, 51, 52, 56, 58, 59, 66 and/or 68. This method is based on amplification with primer pairs and probes labelled with different detectable labels and uses detection in real-time. The method also uses internal control, such as human housekeeping gene. Document US2007/207456 discloses a method for detecting HPV genotypes 16, 18, 31, 33, 35, 39, 45, 51, 52, 56, 58, 66, 69, 6, 11, 34, 40, 42, 43, and 44 and kit therefore. The method uses amplification with primers and the assay is multiplex assay. Moreover, the method uses microarrays/chips with probes and primers that are labelled for the detection of the amplification products.

### Brief description

The present invention relates to a method defined in the appended claims for detecting a human papillomavirus (HPV) genotype associated with development of cervical, oral or anogenital cancer in a subject and for determining the genotype or genotypes of human papillomavirus infecting said subject when said human papillomavirus genotype associated with development of cervical, oral or anogenital cancer is detected therein, comprising:
(a) subjecting at least one sequence comprised in DNA present in a biological sample obtained from said subject to multiplex real-time PCR, wherein said DNA is extracted from said biological sample prior to subjecting it to multiplex real-time PCR, wherein said multiplex real-time PCR amplifies:
   (i) at least one first sequence comprised in said DNA, wherein each first sequence is a sequence of a marker of a genotype of the human papillomavirus, wherein amplification of each sequence is performed using a forward primer and a reverse primer, each complementary to and specific for the locus of said marker; and
   (ii) a second sequence comprised in said DNA, wherein said second sequence is a sequence of a marker of a human housekeeping gene, wherein amplification of said second sequence is performed using a forward primer and a reverse primer, each complementary to and specific for the locus of said marker,

in a composition comprising:
   (iii) a set of probes comprising:
      - a probe comprising a first fluorophore and a sequence, wherein said sequence is complementary to and specific for a sequence of a marker of the 16 genotype of the human papillomavirus;
      - a probe comprising a second fluorophore and a sequence, wherein said sequence is complementary to and specific for a sequence of a marker of the 18 genotype of the human papillomavirus; and
      - a pool of probes, wherein each probe of said pool of probes comprises a third fluorophore and a sequence complementary to and specific for a sequence of a marker of a genotype selected from the 31, 33, 35, 39, 45, 51, 52, 56, 58, 59, 66 and 68 genotypes of the human papillomavirus, wherein each of the 31, 33, 35, 39, 45, 51, 52, 56, 58, 59, 66 and 68 genotypes of the human papillomavirus has a marker comprising a sequence which is complementary to and specific for at least one sequence of a probe of said pool of probes; and
   (iv) a probe comprising a fourth fluorophore and a sequence, wherein said sequence is complementary to and specific for a sequence of a marker of said human housekeeping gene,
wherein each probe that binds to the sequence of a marker complementary thereto and specific therefore is detected,
wherein when:
   - the fourth fluorophore of the probe binding to said second sequence is not detected and the first or second or third fluorophores of the probes binding to a first sequence are not detected, step (a) is repeated for said subject;
   - the fourth fluorophore of the probe binding to said second sequence is detected and at least one of the first or second fluorophores of the probes binding to a first sequence is detected, a human papillomavirus genotype associated with development of cervical, oral or anogenital cancer is detected;
   - the fourth fluorophore of the probe binding to said second sequence is detected and the third fluorophore of the probes of said pool of probes binding to a first sequence is detected, a human papillomavirus genotype associated with development of cervical, oral or anogenital cancer is detected and the composition comprising the amplified first sequence and second sequence is selected;
(b) subjecting each amplified first sequence and second sequence of the composition selected in step (a) to hybridization on a solid support comprising probes attached thereto, wherein during hybridization, the composition selected in step (a) is subjected to heating to at least 60 °C and subsequently, to cooling, wherein each probe comprises a sequence complementary to and specific for a sequence of a marker for which a forward primer and a reverse primer complementary to and specific for the locus of said marker were provided in said multiplex real-time PCR in step (a),
wherein each probe comprising the same sequence is attached to a specific area of said solid support and each specific area of said solid support is attached to probes comprising the same sequence; and
(c) identifying each specific area of said solid support to which a probe is attached and to which a first sequence or second sequence is hybridized;
wherein when:
   (I) the fourth fluorophore of the probe binding to said second sequence is detected and the first fluorophore of the probes binding to a first sequence is detected in step (a), the human papillomavirus genotype associated with development of cervical, oral or anogenital cancer which is detected is determined to be the 16 genotype of the human papillomavirus;
   (II) the fourth fluorophore of the probe binding to said second sequence is detected and the second fluorophore of the probes binding to a first sequence is detected in step (a), the human papillomavirus genotype associated with development of cervical, oral or anogenital cancer which is detected is determined to be the 18 genotype of the human papillomavirus;
   (III) the fourth fluorophore of the probe binding to said second sequence is detected and the third fluorophore of the probes binding to a first sequence is detected in step (a) and at least one specific area of said solid support to which a probe is attached and to which a first sequence is hybridized is identified in step (c), said genotype is determined by:
   (Illa) identifying each specific area of said solid support to which a probe is attached and to which a first sequence is hybridized;
   (Illb) determining which genotype of the human papillomavirus is identified by a marker comprising a sequence complementary to and specific for a sequence of the probe which is attached to each specific area identified in step (IIIa).

In addition, the present invention relates to a use of the system defined in the appended claims for detecting a human papillomavirus genotype associated with development of cervical, oral or anogenital cancer in a subject and for determining the genotype or genotypes of human papillomavirus infecting said subject when said human papillomavirus genotype associated with development of cervical, oral or anogenital cancer is detected therein, comprising:
(a) means for subjecting at least one sequence comprised in DNA present in a biological sample obtained from said subject to multiplex real-time PCR, wherein said multiplex real-time PCR amplifies:
   (i) at least one first sequence comprised in said DNA, wherein each first sequence is a sequence of a marker of a genotype of the human papillomavirus, wherein amplification of each sequence is performed using a forward primer and a reverse primer, each complementary to and specific for the locus of said marker; and
   (ii) a second sequence comprised in said DNA, wherein said second sequence is a sequence of a marker of a human housekeeping gene, wherein amplification of said second sequence is performed using a forward primer and a reverse primer, each complementary to and specific for the locus of said marker,

in a composition comprising:
   (iii) a set of probes comprising:
      - a probe comprising a first fluorophore and a sequence, wherein said sequence is complementary to and specific for a sequence of a marker of the 16 genotype of the human papillomavirus;
      - a probe comprising a second fluorophore and a sequence, wherein said sequence is complementary to and specific for a sequence of a marker of the 18 genotype of the human papillomavirus; and
      - a pool of probes, wherein each probe of said pool of probes comprises a third fluorophore and a sequence complementary to and specific for a sequence of a marker of a genotype selected from the 31, 33, 35, 39, 45, 51, 52, 56, 58, 59, 66 and 68 genotypes of the human papillomavirus, wherein each of the 31, 33, 35, 39, 45, 51, 52, 56, 58, 59, 66 and 68 genotypes of the human papillomavirus has a marker comprising a sequence which is complementary to and specific for at least one sequence of a probe of said pool of probes; and
   (iv) a probe comprising a fourth fluorophore and a sequence, wherein said sequence is complementary to and specific for a sequence of a marker of said human housekeeping gene,
wherein each probe that binds to the sequence of a marker complementary thereto and specific therefor is detected,
wherein when:
   - the fourth fluorophore of the probe binding to said second sequence is not detected and the first or second or third fluorophores of the probes binding to a first sequence are not detected, (a) is repeated for said subject;
   - the fourth fluorophore of the probe binding to said second sequence is detected and at least one of the first or second fluorophores of the probes binding to a first sequence is detected, a human papillomavirus genotype associated with development of cervical, oral or anogenital cancer is detected;
   - the fourth fluorophore of the probe binding to said second sequence is detected and the third fluorophore of the probes of said pool of probes binding to a first sequence is detected, a human papillomavirus genotype associated with development of cervical, oral or anogenital cancer is detected and the composition comprising the amplified first sequence and second sequence is selected, and
   wherein the means for subjecting at least one sequence comprised in DNA present in said biological sample to multiplex real-time PCR comprise a multiplex real-time PCR instrument and multiplex real-time PCR reagents;
(b) means for subjecting each amplified first sequence and second sequence of the composition selected in (a), to hybridization on a solid support comprising probes attached thereto, means for heating the composition selected in (a) to at least 60 °C during hybridization, and means for cooling the composition selected in (a) during hybridization, wherein each probe comprises a sequence complementary to and specific for a sequence of a marker for which a forward primer and a reverse primer complementary to and specific for the locus of said marker were provided in said multiplex real-time PCR in (a),
wherein each probe comprising the same sequence is attached to a specific area of said solid support and each specific area of said solid support is attached to probes comprising the same sequence,
wherein the means for heating the composition selected in (a) comprise a heating device,
wherein the means for cooling the composition selected in (a) comprise a cooling device,
wherein the means for subjecting each first sequence and second sequence which is amplified in the composition selected in (a) to hybridization on a solid support comprise a solid support comprising probes attached thereto, wherein each probe comprises a sequence complementary to and specific for a sequence of a marker for which a forward primer and a reverse primer complementary to and specific for the locus of said marker were provided in said multiplex real-time PCR in (a), and hybridization reagents; and
(c) means for identifying each specific area of said solid support to which a probe is attached and to which a first sequence or second sequence is hybridized, wherein said means comprise an image-capture device;
wherein when:
   (I) the fourth fluorophore of the probe binding to said second sequence is detected and the first fluorophore of the probes binding to a first sequence is detected in (a), the human papillomavirus genotype associated with development of cervical, oral or anogenital cancer which is detected is determined to be the 16 genotype of the human papillomavirus;
   (II) the fourth fluorophore of the probe binding to said second sequence is detected and the second fluorophore of the probes binding to a first sequence is detected in (a), the human papillomavirus genotype associated with development of cervical, oral or anogenital cancer which is detected is determined to be the 18 genotype of the human papillomavirus;
   (III) the fourth fluorophore of the probe binding to said second sequence is detected and the third fluorophore of the probes binding to a first sequence is detected in (a) and at least one specific area of said solid support to which a probe is attached and to which a first sequence is hybridized is identified in (c), said genotype is determined by:
      (IIIa) identifying each specific area of said solid support to which a probe is attached and to which a first sequence is hybridized;
      (Illb) determining which genotype of the human papillomavirus is identified by a marker comprising a sequence complementary to and specific for a sequence of the probe which is attached to each specific area identified in (IIIa).

The system may optionally further comprise means for extracting DNA from a biological sample such as means for extracting DNA of human papillomavirus present in a biological sample obtained from a subject. Means for extracting DNA can be used before using means for subjecting at least one sequence comprised in DNA present in a biological sample obtained from said subject to multiplex real-time PCR,

Furthermore, the present invention relates to a use of a kit defined in the appended claims for detecting a human papillomavirus genotype associated with development of cervical, oral or anogenital cancer in a subject and for determining the genotype or genotypes of human papillomavirus infecting said subject when said human papillomavirus genotype associated with development of cervical, oral or anogenital cancer is detected therein, comprising:
(a) means for subjecting at least one sequence comprised in DNA present in a biological sample obtained from said subject to multiplex real-time PCR, wherein said multiplex real-time PCR amplifies:
   (i) at least one first sequence comprised in said DNA, wherein each first sequence is a sequence of a marker of a genotype of the human papillomavirus, wherein amplification of each sequence is performed using a forward primer and a reverse primer, each complementary to and specific for the locus of said marker; and
   (ii) a second sequence comprised in said DNA, wherein said second sequence is a sequence of a marker of a human housekeeping gene, wherein amplification of said second sequence is performed using a forward primer and a reverse primer, each complementary to and specific for the locus of said marker,

in a composition comprising:
   (iii) a set of probes comprising:
      - a probe comprising a first fluorophore and a sequence, wherein said sequence is complementary to and specific for a sequence of a marker of the 16 genotype of the human papillomavirus;
      - a probe comprising a second fluorophore and a sequence, wherein said sequence is complementary to and specific for a sequence of a marker of the 18 genotype of the human papillomavirus; and
      - a pool of probes, wherein each probe of said pool of probes comprises a third fluorophore and a sequence complementary to and specific for a sequence of a marker of a genotype selected from the 31, 33, 35, 39, 45, 51, 52, 56, 58, 59, 66 and 68 genotypes of the human papillomavirus, wherein each of the 31, 33, 35, 39, 45, 51, 52, 56, 58, 59, 66 and 68 genotypes of the human papillomavirus has a marker comprising a sequence which is complementary to and specific for at least one sequence of a probe of said pool of probes; and
   (iv) a probe comprising a fourth fluorophore and a sequence, wherein said sequence is complementary to and specific for a sequence of a marker of said human housekeeping gene,
wherein each probe that binds to the sequence of a marker complementary thereto and specific therefor is detected,
wherein when:
   - the fourth fluorophore of the probe binding to said second sequence is not detected and the first or second or third fluorophores of the probes binding to a first sequence are not detected, (a) is repeated for said subject;
   - the fourth fluorophore of the probe binding to said second sequence is detected and at least one of the first or second fluorophores of the probes binding to a first sequence is detected, a human papillomavirus genotype associated with development of cervical, oral or anogenital cancer is detected;
   - the fourth fluorophore of the probe binding to said second sequence is detected and the third fluorophore of the probes of said pool of probes binding to a first sequence is detected, a human papillomavirus genotype associated with development of cervical, oral or anogenital cancer is detected and the composition comprising the amplified first sequence and second sequence is selected, and
   wherein the means for subjecting at least one sequence comprised in DNA present in said biological sample to multiplex real-time PCR comprise a multiplex real-time PCR instrument and multiplex real-time PCR reagents;
(b) means for subjecting each amplified first sequence and second sequence of the composition selected in (a), to hybridization on a solid support comprising probes attached thereto, means for heating the composition selected in (a) to at least 60 °C during hybridization, and means for cooling the composition selected in (a) during hybridization, wherein each probe comprises a sequence complementary to and specific for a sequence of a marker for which a forward primer and a reverse primer complementary to and specific for the locus of said marker were provided in said multiplex real-time PCR in (a),
wherein each probe comprising the same sequence is attached to a specific area of said solid support and each specific area of said solid support is attached to probes comprising the same sequence,
wherein the means for heating the composition selected in (a) comprise a heating device,
wherein the means for cooling the composition selected in (a) comprise a cooling device,
wherein the means for subjecting each first sequence and second sequence which is amplified in the composition selected in (a) to hybridization on a solid support comprise a solid support comprising probes attached thereto, wherein each probe comprises a sequence complementary to and specific for a sequence of a marker for which a forward primer and a reverse primer complementary to and specific for the locus of said marker were provided in said multiplex real-time PCR in (a), and hybridization reagents; and
(c) means for identifying each specific area of said solid support to which a probe is attached and to which a first sequence or second sequence is hybridized, wherein said means comprise an image-capture device;
wherein when:
   (I) the fourth fluorophore of the probe binding to said second sequence is detected and the first fluorophore of the probes binding to a first sequence is detected in (a), the human papillomavirus genotype associated with development of cervical, oral or anogenital cancer which is detected is determined to be the 16 genotype of the human papillomavirus;
   (II) the fourth fluorophore of the probe binding to said second sequence is detected and the second fluorophore of the probes binding to a first sequence is detected in (a), the human papillomavirus genotype associated with development of cervical, oral or anogenital cancer which is detected is determined to be the 18 genotype of the human papillomavirus;
   (III) the fourth fluorophore of the probe binding to said second sequence is detected and the third fluorophore of the probes binding to a first sequence is detected in (a) and at least one specific area of said solid support to which a probe is attached and to which a first sequence is hybridized is identified in (c), said genotype is determined by:
      (IIIa) identifying each specific area of said solid support to which a probe is attached and to which a first sequence is hybridized;
      (Illb) determining which genotype of the human papillomavirus is identified by a marker comprising a sequence complementary to and specific for a sequence of the probe which is attached to each specific area identified in (IIIa).

The kit may optionally further comprise means for extracting DNA from a biological sample such as means for extracting DNA of human papillomavirus present in a biological sample obtained from a subject. Means for extracting DNA can be used before using means for subjecting at least one sequence comprised in DNA present in a biological sample obtained from said subject to multiplex real-time PCR,

The present invention also is characterised by a use of a forward and/or reverse primer for obtaining an amplified sequence comprising a molecular tag by multiplex real-time PCR, wherein said multiplex real-time PCR amplifies a sequence comprised in DNA present in a biological sample obtained from a subject, wherein said DNA is extracted from said biological sample prior to subjecting it to multiplex real-time PCR,
wherein said forward and/or reverse primer comprises
   - a sequence complementary to and specific for one or more locus of a marker of the genotype 16, 18, 31, 33, 35, 39, 45, 51, 52, 56, 58, 59, 66 and/or 68 of the human papillomavirus; and
   - a molecular tag; and
for identifying a specific area on a solid support to which a probe is attached, wherein said probe comprises a sequence complementary to and specific for a sequence of a marker for which said forward and/or reverse primer is complementary to and specific for; and to which said amplified sequence comprising a molecular tag is hybridized.

The present invention also is characterised by a use of an amplified sequence comprising a molecular tag,
wherein said amplified sequence has been obtained in multiplex real-time PCR, wherein said multiplex real-time PCR amplifies a sequence comprised in DNA present in a biological sample obtained from a subject, wherein said DNA is extracted from said biological sample prior to subjecting it to multiplex real-time PCR,
wherein amplification is performed using a forward and/or reverse primer, wherein said forward and/or reverse primer comprises
   - a sequence complementary to and specific for one or more locus of a marker of the genotype 16, 18, 31, 33, 35, 39, 45, 51, 52, 56, 58, 59, 66 and/or 68 of the human papillomavirus; and
   - a molecular tag; and
for identifying a specific area on a solid support to which a probe is attached, wherein said probe comprises a sequence complementary to and specific for a sequence of a marker for which said forward and/or reverse primer is complementary to and specific for; and to which said amplified sequence comprising a molecular tag is hybridized.

### Description of the Figures

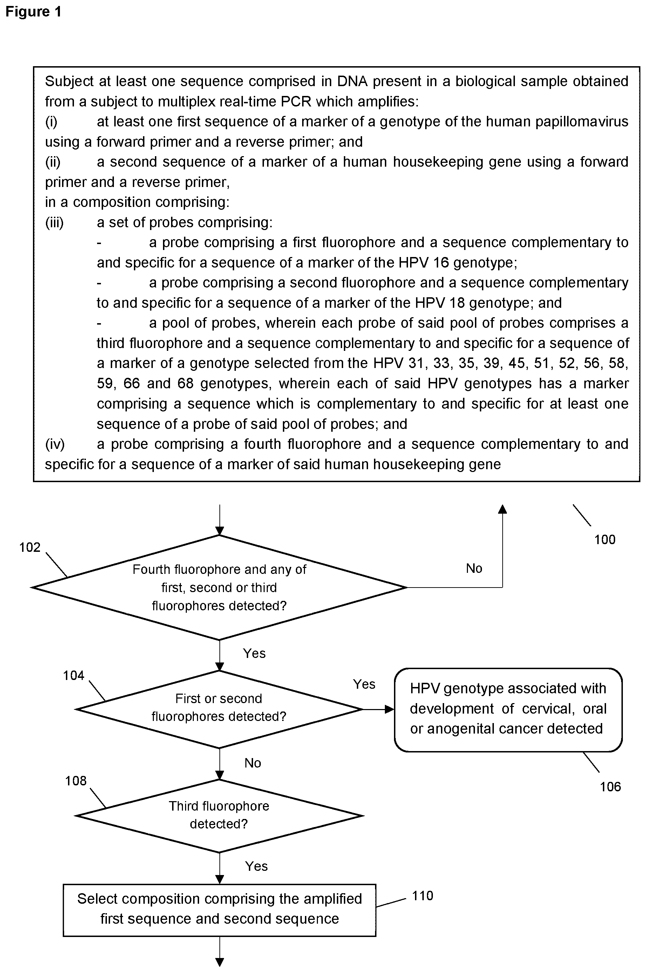

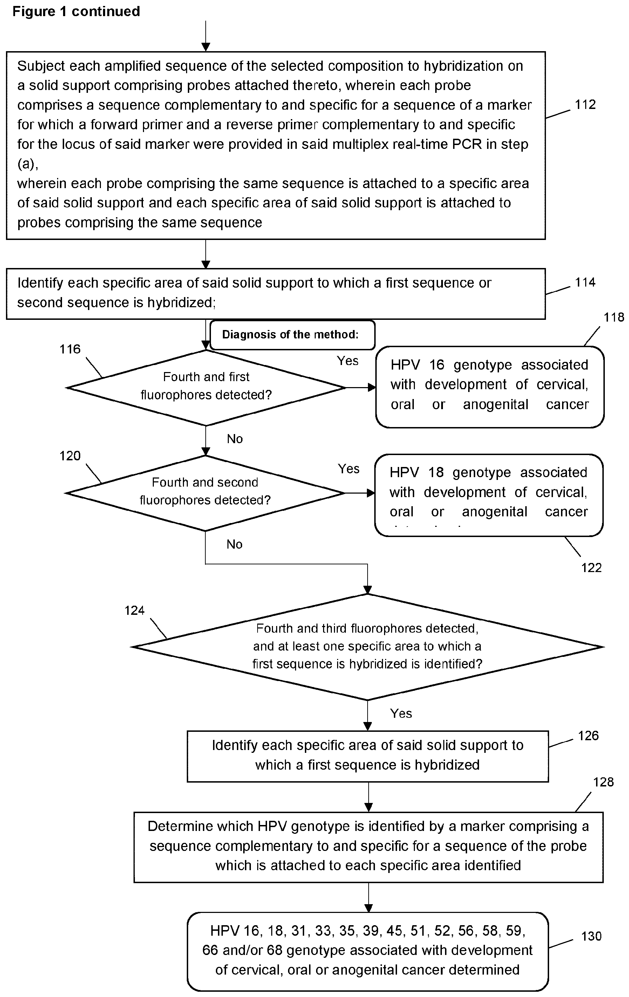

**Figure 1****:** Flow chart of an example of the method for detecting a human papillomavirus genotype associated with development of cervical, oral or anogenital cancer in a subject and for determining the genotype or genotypes of human papillomavirus infecting said subject when said human papillomavirus genotype associated with development of cervical, oral or anogenital cancer is detected therein, in accordance with the present invention.

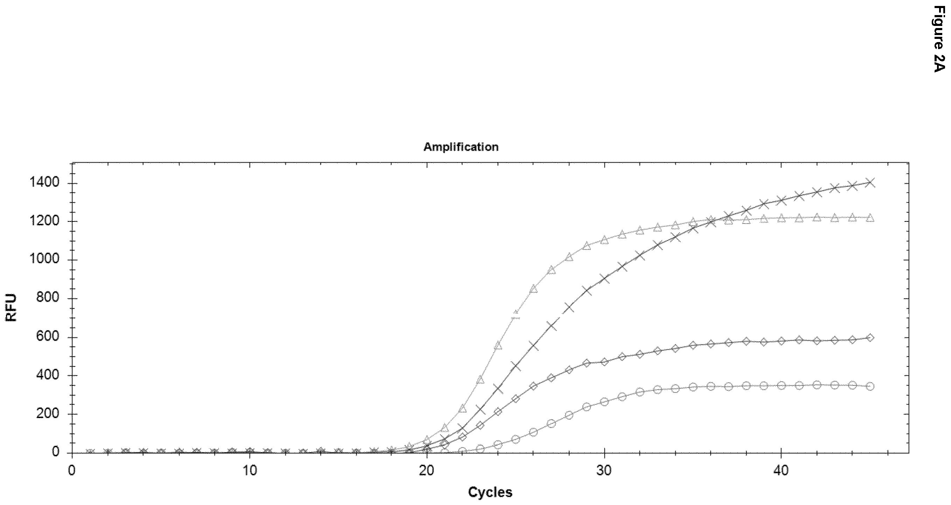

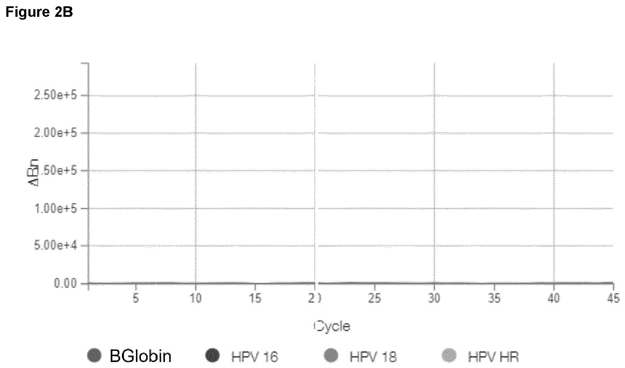

**Figure 2****:** Graphs of amplification of **A.** the positive control (PC) and **B.** a negative control with water (NTC) according to the multiplex real-time PCR procedure of (a) of the present invention, with expected count values (ΔRn) over time (per multiplex PCR cycle) for PC: HPV 16 (ROX) 20±2; HPV 18 (Cy5) 20±2; HPV HR (31, 33, 35, 39, 45, 51, 52, 56, 58, 59, 66 and 68 genotypes of HPV, FAM) 20±2, Bglobin (human beta globin, JOE) 20±2. Experiment performed in Applied Biosystems QuantStudio^{™} 5 Real-Time PCR System. (O) beta globin; (0) HPV HR, (Δ) HPV 16; (x)HPV 18.

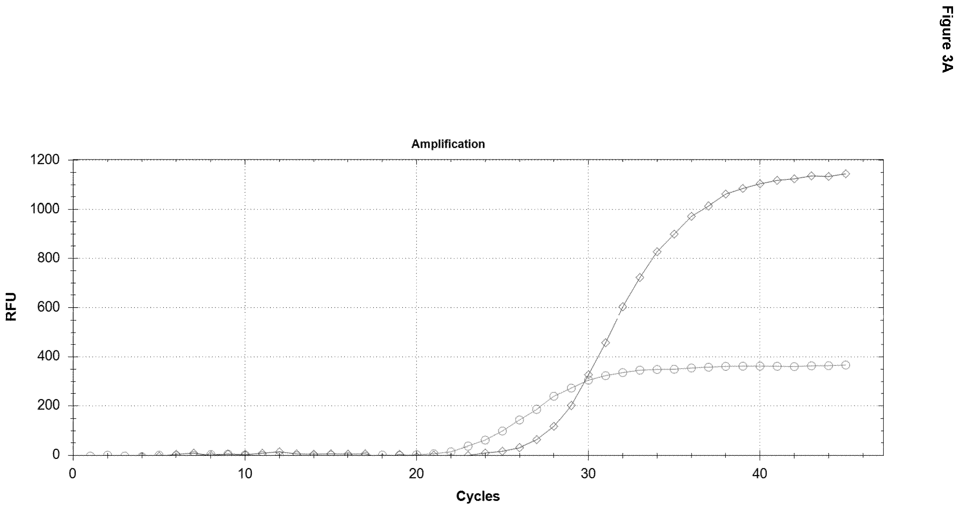

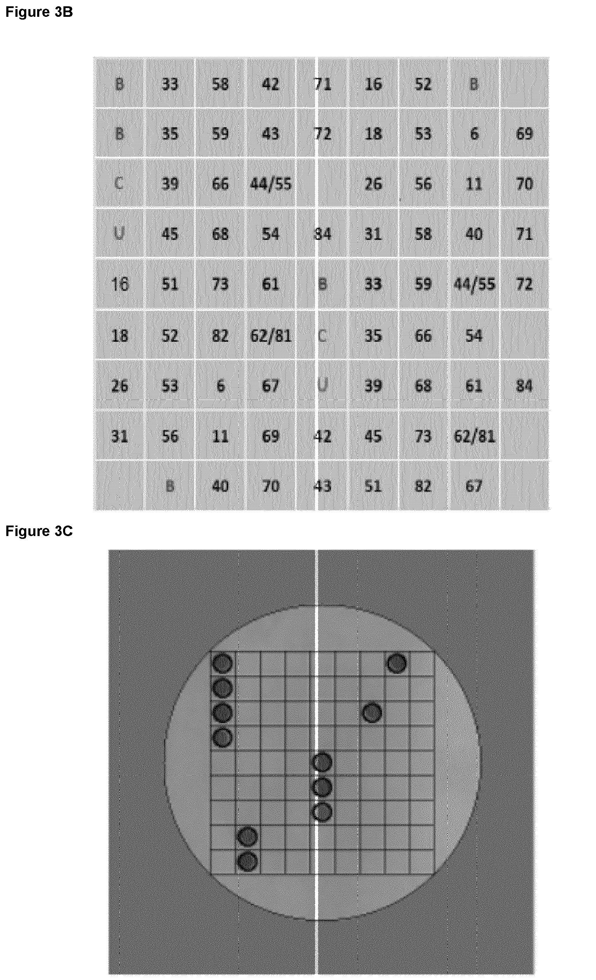

**Figure 3****:** Detection and determination of genotype 56 of human papillomavirus in a subject infected therewith based on **A.** Graphs of amplification of beta globulin and HPV HR according to the multiplex real-time PCR procedure of (b) of the present invention, with expected count values (ΔRn) over time (per multiplex PCR cycle) for PC: HPV HR (31, 33, 35, 39, 45, 51, 52, 56, 58, 59, 66 and 68 genotypes of HPV, FAM) 20±2, Bglobin (human beta globin, JOE) 20±2. Experiment performed in Applied Biosystems QuantStudio^{™} 5 Real-Time PCR System. (O) beta globin; (0) HPV HR. **B.** visualisation image following hybridization to a solid support of amplicons [obtained by multiplex real-time PCR according to (a) of the present invention] of a sample from said subject, wherein the specific areas 2H and 7C (specific areas are identified by a numeral and letter of the alphabet, respectively, counted from the top left specific area 1A of said solid support) show hybridization has taken place, said specific areas corresponding to those of said solid support to which a probe comprising a sequence complementary to and specific for a sequence of a marker of the HPV 56 genotype is attached, as shown in **C.** the schematic of said solid support. Additional hybridization has taken place at the specific areas (1,A; 1,B; 2,I; 5,E and 8,A) of said solid support to which a probe is attached for use as a hybridization control (5 specific areas serve to correctly orient the solid support), at the specific areas (1,C and 5,F) of said solid support to which a probe is attached for use as an internal (human genomic) DNA control, and at the specific areas (1,D and 5,G) of said solid support to which multiple probes are attached for use as a degenerated universal HPV control. Specific areas 1,I; 5,C; 9,A; 9,F; 9;H and 9,I have no probe attached thereto, while remaining specific areas have probes attached thereto, wherein each probe is complementary for and specific to a sequence of a marker of the genotype(s) having the number(s) indicated in said schematic, each specific area of a given probe type being provided in duplicate. Note that specific areas 4,C; 4,F; 8,E and 8,H are specific areas where the probe attached thereto is complementary for and specific to a sequence of a marker of two genotypes.

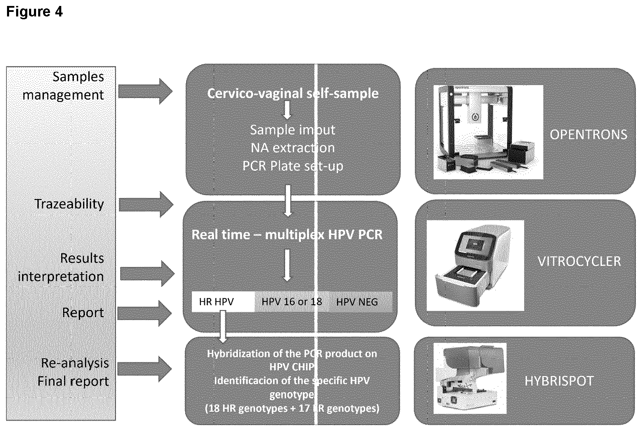

**Figure 4****:** Further detailed flow chart of an example of the method for detecting a human papillomavirus genotype associated with development of cervical, oral or anogenital cancer in a subject and for determining the genotype or genotypes of human papillomavirus infecting said subject when said human papillomavirus genotype associated with development of cervical, oral or anogenital cancer is detected therein, in accordance with the present invention.

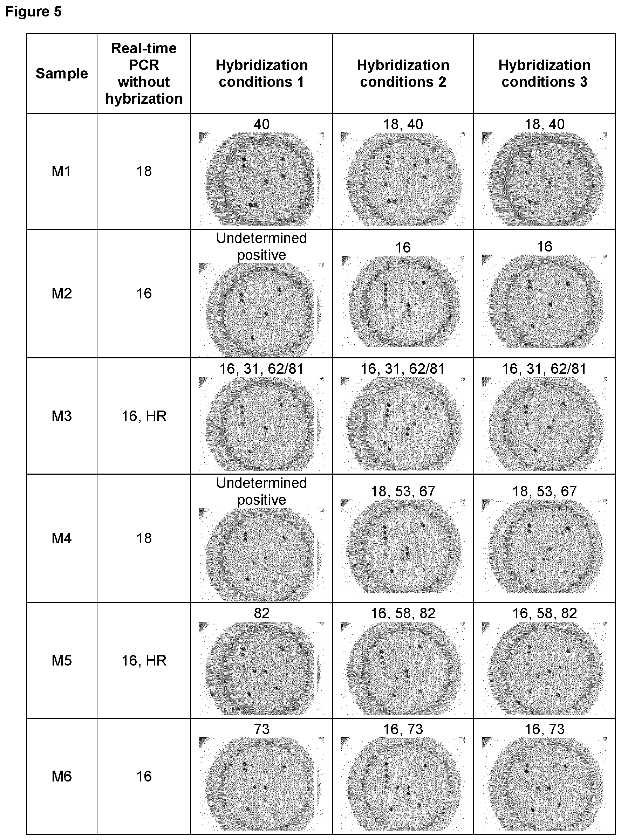

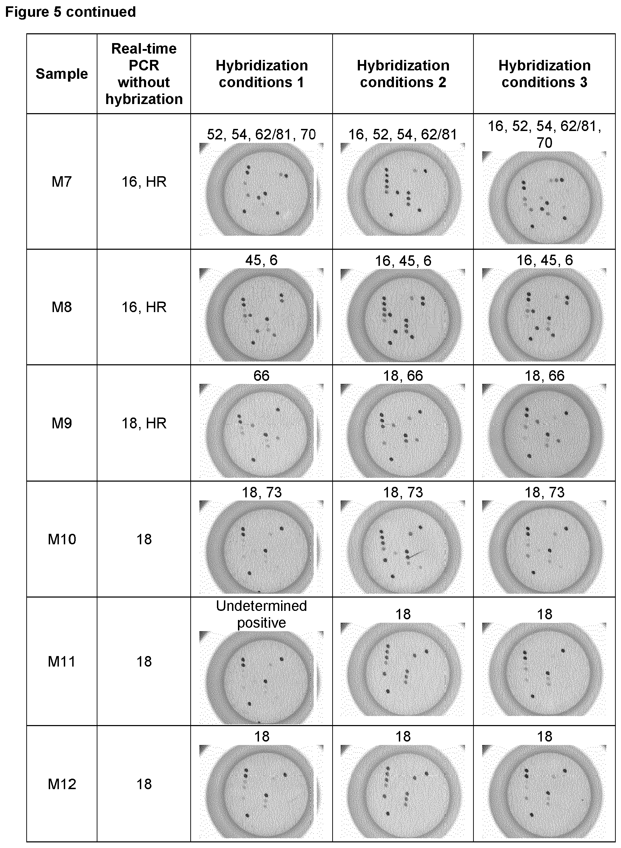

**Figure 5****:** Detection and determination of human papillomavirus in subjects infected therewith using different hybridization conditions. The same group of samples (liquid cytology samples with previous positive results for HR HPV genotypes by real-time PCR) was assayed with different hybridization conditions. Hybridization conditions 1 correspond to real-time PCR, followed by hybridization of the PCR product at a temperature of 41°C. Under these conditions, when the detected signal is only provided by the universal HPV probe, an undetermined genotype of HPV in the sample is assessed (marked as "undetermined positive" in the figure). Hybridization conditions 2 correspond to amplification of the samples by an end point PCR without fluorescent probes and subsequent hybridization of the PCR product at a temperature of 41 °C. Hybridization conditions 3 correspond to real-time PCR, followed by hybridization at a temperature of 60 °C, and subsequent cooling to 41 °C, according to the method of the invention.

### Detailed description

The present invention relates to a method, use of a system and use of a kit for detecting a human papillomavirus genotype associated with development of cervical, oral or anogenital cancer in a subject and for determining the genotype or genotypes of human papillomavirus infecting said subject when said human papillomavirus genotype associated with development of cervical, oral or anogenital cancer is detected therein. Thus, said method, said uses of the system and the kit are for detecting a human papillomavirus genotype associated with development of cervical, oral or anogenital cancer in a subject and for determining the genotype or genotypes thereof when detected.

Said human papillomavirus genotype associated with development of cervical, oral or anogenital cancer in a subject is a genotype of a human DNA virus from the *Papillomaviridae* family. Said human papillomavirus genotype is associated with development of cervical, oral or anogenital cancer in a subject, and therefore is a human papillomavirus genotype which may cause, increase, promote or otherwise result in development of cervical, oral or anogenital cancer, respectively, in said subject when infected therewith.

Said cervical cancer is cancer of the lower part of the uterus, preferably cancer of the squamocolumnar junction of the cervix, more preferably a squamous cell carcinoma (epidermoid carcinoma) of the squamocolumnar junction of the cervix.

Said oral cancer can be cancer of the oral cavity and/or pharynx. It includes cancer of mouth, tonsils and/or throat. The oral cancer may be an oropharyngeal cancer.

Said anogenital cancer comprises cancer of the external sex organs and/or anal cancer. Said cancer of the external sex organs preferably is selected from the group consisting of cancer of the vulva, vagina, penis, scrotum and testicles, and any anatomical parts thereof. More preferably said cancer of the external sex organs is selected from the group consisting of cancer of the urethra, hymen, clitoral frenulum, clitoral glans, clitoral hood, labia majora, labia minora, frenulum of labia minora, Skene's gland, vagina, foreskin, frenulum of penis, glans penis, scrotum and testicles, and any anatomical parts thereof. Even more preferably said cancer of the external sex organs is selected from the group consisting of a squamous cell carcinoma (epidermoid carcinoma), an adenocarcinoma, a small cell carcinoma, a lymphoma, a sarcoma and a melanoma, yet more preferably a squamous cell carcinoma in any of the aforementioned external sex organ tissues.

Thus, the anogenital cancer can be an anal cancer, a vaginal cancer, a vulvar cancer and/or a penile cancer.

Said anal cancer preferably is selected from the group consisting of cancer of the anal canal, anal columns of Morgagni, squamocolumnar junction, ischioanal fossa, perianal space, anal verge, anal crypt, anal glands and anal sphincter. More preferably said anal cancer is selected from the group consisting of a squamous cell carcinoma (epidermoid carcinoma), an adenocarcinoma, a small cell carcinoma, a lymphoma, a sarcoma and a melanoma, even more preferably a squamous cell carcinoma in any of the aforementioned anal tissues, yet more preferably a squamous cell carcinoma of the squamocolumnar junction.

Said subject is a human subject, preferably a male or female human subject, more preferably a sexually active male subject or a sexually active female human subject.

A human papillomavirus genotype associated with development of cervical, oral or anogenital cancer in a subject infected therewith is a genotype that causes a subject infected therewith to be at high risk of development of cervical, oral or anogenital cancer. Preferably, said human papillomavirus genotype associated with development of cervical, oral or anogenital cancer in a subject infected therewith is selected from the group consisting of the 16, 18, 31, 33, 35, 39, 45, 51, 52, 56, 58, 59, 66 and 68 genotypes of the human papillomavirus, as also disclosed in other aspects of the invention described herein. The method, use of the system and use of the kit of the present invention may detect one or more of such genotypes in any given subject infected therewith.

In contrast, a human papillomavirus genotype not associated with development of cervical, oral or anogenital cancer in a subject infected therewith is a genotype that causes a subject infected therewith to be at low risk of development of cervical, oral or anogenital cancer. Preferably, said human papillomavirus genotype not associated with development of cervical, oral or anogenital cancer in a subject infected therewith is additionally selected from the group consisting of the 6, 11, 26, 40, 42, 43, 44, 53, 54, 55, 61, 62, 67, 69, 70, 71, 72, 73, 81, 82 and 84 genotypes of the human papillomavirus. However, said human papillomavirus genotypes not associated with development of cervical, oral or anogenital cancer in a subject infected therewith may nevertheless infect a subject who is infected with one or more human papillomavirus genotypes that are associated with development of cervical, oral or anogenital cancer.

Consequently, in other words, the method, the uses of the system and the kit of the present invention is for detecting one or more genotypes associated with development of cervical, oral or anogenital cancer and for determining the genotype or genotypes of human papillomavirus associated with development of cervical, oral or anogenital cancer which is or are detected therein, and, optionally, one or more genotypes not associated with development of cervical, oral or anogenital cancer, in a subject infected therewith. In an especially preferred embodiment of the method, the use of the system and the use of the kit of the present invention is for detecting one or more genotypes associated with development of cervical, oral or anogenital cancer and for determining the genotype or genotypes of human papillomavirus associated with development of cervical, oral or anogenital cancer which is or are detected therein, and, optionally, one or more genotypes not associated with development of cervical, oral or anogenital cancer, in a subject infected therewith, wherein each genotype associated with development of cervical, oral or anogenital cancer is selected from the group consisting of the 31, 33, 35, 39, 45, 51, 52, 56, 58, 59, 66 and 68 genotypes of the human papillomavirus.

The present invention comprises two techniques, namely multiplex real-time PCR [described by step (a) in the method of the present invention and by means (a) in the uses of system and kit of the present invention], and hybridisation plus visualisation [respectively described by steps (b) plus (c) in the method of the present invention and by means (b) plus (c) in the uses of system and kit of the present invention]. Multiplex PCR comprises the simultaneous detection of multiple specific nucleotide sequences (*i.e*. genetic markers) in a single composition (*i.e*. PCR reaction well or vessel), upon amplification by PCR using a different pair of primers for each marker. Multiplex real-time PCR therefore comprises said simultaneous detection in "real time" (*i.e*. at predetermined intervals), preferably after each amplification cycle of said PCR.

Thus, said (a) in the method of the present invention comprises a step of subjecting at least one sequence comprised in DNA present in a biological sample obtained from said subject to multiplex real-time PCR (block 100 of **Figure 1****),** while said (a) in the uses of the system and kit of the present invention comprises means for subjecting at least one sequence comprised in DNA present in a biological sample obtained from said subject to multiplex real-time PCR.

The PCR product added to the solid support contained the probes remaining from the real-time PCR that have not been exhausted during amplification, so there will be two complementary probes in the hybridization device, one in liquid phase and the other in solid phase, and both could compete to hybridize with the amplicon of interest. This is prevented by heating at a temperature of at least 60 °C, which promotes that the generated amplicon hybridizes preferably with the solid-phase probe, i.e. the one in the array.

Thus, in the method, uses of system and kit of the present invention, the composition selected in (a) is subjected to heating to at least 60 °C, and gradually cooling while hybridizing to the solid surface according to step (b).

Heating at a temperature of at least 60 °C promotes that the generated amplicon hybridizes preferentially with the probes in the solid phase, and not to probes remaining from the real-time PCR in the liquid phase, improving the sensitivity and specificity of the method. If the heating temperature is below 60 °C (for example, 50 °C or 41 °C, as assayed in Example 4), some of the probes remaining from the PCR could hybridize to the amplicon of interest and prevent it from binding to the probe in the solid support, as shown in Example 4.

Example 4 shows that when the real-time PCR is followed by hybridization at a temperature of 60 °C, according to the method of the invention, the genotypes present in the sample are properly detected and identified, consequently, the sensitivity and specificity of the method are improved. When the real-time PCR is followed by hybridization of the real-time PCR products at a temperature of 41°C, as assayed in Example 4, some genotypes are not identified.

Said biological sample is a sample comprising biological fluid, free cells and/or tissues obtained from said subject. Said sample may be obtained by a third party (e.g. doctor, nurse) or by the subject themselves. In a preferred embodiment of the present invention, the biological sample obtained from said subject was obtained by the subject obtaining said sample by themselves. For example, the biological sample from the cervix obtained from said subject may have been obtained by the subject obtaining said sample by themselves. Said sample is preferably obtained by means of a sample collection device such as a swab, brush or veil device applied to the cervical, oral or anogenital tissue, preferably cervical tissue of said subject, so as to obtain biological fluid and/or cells from said subject. The sample collection device may thus be a swab, brush, or a vaginal veil device.

Said biological sample comprises DNA. Said DNA is a polynucleotide and therefore comprises at least one sequence of nucleotides. If said subject is infected with a human papillomavirus associated with development of cervical, oral or anogenital cancer, said biological sample will comprise DNA from said human papillomavirus. If said subject is not infected with a human papillomavirus associated with development of cervical, oral or anogenital cancer, said biological sample will comprise DNA from other sources, namely DNA from the subject and/or DNA from a human papillomavirus not associated with development of cervical, oral or anogenital cancer.

DNA is usually extracted from the sample in order to carry out said multiplex real-time PCR amplification. Thus, said amplification can be performed from purified DNA samples.

Said PCR (*i.e*. multiplex real-time PCR) amplifies:
(i) at least one first sequence comprised in said DNA, wherein each first sequence is a sequence of a marker of a genotype of the human papillomavirus, wherein amplification of each sequence is performed using a forward primer and a reverse primer, each complementary to and specific for the locus of said marker; and
(ii) a second sequence comprised in said DNA, wherein said second sequence is a sequence of a marker of a human housekeeping gene, wherein amplification of said second sequence is performed using a forward primer and a reverse primer, each complementary to and specific for the locus of said marker (block 100 of **Figure 1****).**

Each amplification is performed by said PCR using a pair of primers, whereby said pair of primers comprises a locus-specific forward primer and a locus-specific reverse primer which bind to different complementary sequences on the Watson and Crick strands adjacent to said nucleotide sequence, thereby identifying the 5' and 3' limits of said nucleotide sequence. In particular, the 3' end of the nucleotide sequence of the Watson strand begins with the nucleotide which is adjacent to the nucleotide at the 5' end of the sequence that is annealed with the forward primer. Conversely, the 5' end of the nucleotide sequence of the Watson strand begins with the nucleotide complementary to the nucleotide which is adjacent to the nucleotide at the 5' end of the sequence that is annealed with the reverse primer. Likewise, the 3' end of the nucleotide sequence of the Crick strand begins with the nucleotide which is adjacent to the nucleotide at the 5' end of the sequence that is annealed with the reverse primer. Conversely, the 5' end of the nucleotide sequence of the Crick strand begins with the nucleotide complementary to the nucleotide which is adjacent to the nucleotide at the 5' end of the sequence that is annealed with the forward primer. Accordingly, a DNA polymerase attaches to the 5' end of the aforementioned primers and replicates the nucleotide sequence multiple times.

The primers are locus-specific primers chosen so as to be complementary to and therefore identify a specific nucleotide sequence (*i.e*. a genetic marker) that may be present in the biological sample, wherein said marker is:
- indicative of said human papillomavirus (*i.e.* the presence of human papillomavirus DNA in said sample and, hence, infection of said subject with said human papillomavirus) in the case of (i), and
- indicative of said human housekeeping gene (*i.e.* the presence of human DNA from said subject in said sample) in the case of (ii).

Thus, the method, uses of the system and kit of the present invention relies on any genetic marker of a human papillomavirus and of a human housekeeping gene that is detectable using an amplicon sequencing approach.

Since said multiplex real-time PCR amplifies at least one first sequence (of a marker of a genotype of the human papillomavirus) and a second sequence (of a marker of a genotype of a human housekeeping gene), and amplification of each sequence is performed using a forward primer and a reverse primer, each complementary to and specific for the locus of any given marker, said multiplex real-time PCR is performed using at least two pairs of forward and reverse primers. Preferably, said multiplex real-time PCR is performed using at least two pairs of forward and reverse primers for amplification of first sequences and a pair of forward and reverse primers is used for amplification of a second sequence, such that at least two different first sequences may be amplified by said multiplex real-time PCR, each first sequence being a marker of a genotype of the human papillomavirus, and a second sequence may be amplified by said multiplex real-time PCR, said second sequence being a marker of a genotype of a human housekeeping gene. Any given pair of forward and reverse primers for amplification of first sequences may amplify a marker common to one or more genotypes of the human papillomavirus.

Each first sequence is a sequence of a marker of a genotype of the human papillomavirus, irrespective of whether said human papillomavirus is associated with development of cervical, oral or anogenital cancer or not. In a preferred embodiment of the method, uses, system and kit of the present invention, said multiplex real-time PCR amplifies:
(i) at least one first sequence, wherein each first sequence is a sequence of a marker from a genotype of the human papillomavirus which is present in the conserved region of the *HPV L1* or *HPV L2* gene, more preferably in the conserved region of the *HPV L1* gene; and
(ii) said second sequence.

In another preferred embodiment of the method, uses of system and kit of the present invention, said multiplex real-time PCR additionally amplifies:
(i) at least one first sequence, wherein each first sequence is a sequence of a marker from a genotype of the human papillomavirus which is selected from the 6, 11, 16, 18, 26, 31, 33, 35, 39, 40, 42, 43, 44, 45, 51, 52, 53, 54, 55, 56, 58, 59, 61, 62, 66, 67, 68, 69, 70, 71, 72, 73, 81, 82 and 84 genotypes of the human papillomavirus; and
(ii) said second sequence.

In another more preferred embodiment of the method, uses of system and kit of the present invention, said multiplex real-time PCR amplifies:
(i)
   - a first sequence which is a sequence of a marker from the 16 genotype of the human papillomavirus;
   - a first sequence which is a sequence of a marker from the 18 genotype of the human papillomavirus, and,
   - at least one other first sequence, wherein each first sequence is a sequence of a marker from a genotype of the human papillomavirus which is selected from the 31, 33, 35, 39, 45, 51, 52, 56, 58, 59, 66 and 68 genotypes of the human papillomavirus; and
(ii) said second sequence.

Each second sequence is a sequence of a marker of a genotype of a human housekeeping gene.

Preferably said human housekeeping gene is a constitutive gene that is transcribed continually in said subject, as required for maintenance of cellular function. More preferably, said human housekeeping gene is a human beta globin gene (*HBB* gene) or a ribonuclease P gene (*RNase* P gene), even more preferably a *HBB* gene.

Preferably, said forward and reverse primers for amplification of each first sequence are selected from any of the pairs of sequences of SEQ ID NO:1 to SEQ ID NO:2 disclosed in **Table 1,** while said forward and reverse primers for amplification of said second sequence are selected from any of the pairs of sequences of SEQ ID NO:3 to SEQ ID NO:4 disclosed in **Table 2.**

**Table 1: Pairs of forward and reverse primers for amplification of each first sequence**

| **Gene** | **Sequence (5' - 3')** | **Direction** | **SEQ ID NO** |
|---|---|---|---|
| L1 | *-TTTGTTACTGTGGTAGATACTAC | Forward | SEQ ID NO: 1 |
| | *-GAAAAATAAACTGTAAATCATATTC | Reverse | SEQ ID NO: 2 |

**Table 2: Pairs of forward and reverse primers for amplification of each second sequence**

| **Gene** | **Sequence (5' - 3')** | **Direction** | **SEQ ID NO** |
|---|---|---|---|
| Beta Globin | *-GAGCCATCTATTGCTTACA | Forward | SEQ ID NO: 3 |
| | *-CTCACCACCAACTTCATC | Reverse | SEQ ID NO: 4 |

| | | | |
|---|---|---|---|
| *- represents the 5'-molecular tag of the primer, in this case the molecular tag is biotin. | | | |

The multiplex real-time PCR used in the present invention amplifies DNA from the subject and/or DNA from a human papillomavirus in a composition. Said composition comprises, in addition to said DNA, forward primers and reverse primers and multiple probes, preferably taqman probes. In particular, said multiple probes comprised in said composition comprise:
(iii) a set of probes comprising:
   - a probe comprising a first fluorophore and a sequence, wherein said sequence is complementary to and specific for a sequence of a marker of the 16 genotype of the human papillomavirus;
   - a probe comprising a second fluorophore and a sequence, wherein said sequence is complementary to and specific for a sequence of a marker of the 18 genotype of the human papillomavirus; and
   - a pool of probes, wherein each probe of said pool of probes comprises a third fluorophore and a sequence complementary to and specific for a sequence of a marker of a genotype selected from the 31, 33, 35, 39, 45, 51, 52, 56, 58, 59, 66 and 68 genotypes of the human papillomavirus, wherein each of the 31, 33, 35, 39, 45, 51, 52, 56, 58, 59, 66 and 68 genotypes of the human papillomavirus has a marker comprising a sequence which is complementary to and specific for at least one sequence of a probe of said pool of probes; and
(iv) a probe comprising a fourth fluorophore and a sequence, wherein said sequence is complementary to and specific for a sequence of a marker of said human housekeeping gene (block 100 of **Figure 1****).**

Each probe disclosed in (iii) and (iv) comprises a fluorophore and a sequence, wherein said sequence is complementary to and specific for a sequence of a marker of a gene. When said sequence binds with/anneals to a complementary sequence of a marker of a gene (i.e. for which it is specific) which is amplified by said multiplex PCR, said fluorophore emits light which can be detected. In other words, each probe that binds to the sequence of a marker complementary thereto and specific therefor is detected.

In particular, each probe of the set of probes disclosed in (iii) comprises a fluorophore and a sequence, wherein said sequence is complementary to and specific for a sequence of a marker of the 16, 18, 31, 33, 35, 39, 45, 51, 52, 56, 58, 59, 66 and 68 genotypes of the human papillomavirus. In this way, a sequence of a marker of any of said human papillomavirus genotypes may be detected in the composition subjected to multiplex PCR upon amplification thereof by detection in real-time of the light emitted from the fluorophore of a probe comprising a sequence which is complementary to and specific for said sequence of said marker, when said sequence of said probe binds to said sequence of said marker. Consequently, the method, uses, system and kit of the present invention permit a human papillomavirus having any of said genotypes that may cause a subject infected therewith to be at high risk of development of cervical, oral or anogenital cancer to be detected. Preferably each probe for a given target (HPV genotype or set of HPV genotypes) comprises a sequence selected from any of the sequences shown in **Table 3.**

**Table 3: Sequence of probes used for detection of each first sequence in multiplex real-time PCR**

| **Target** | **Sequence (5' - 3')** | **SEQ ID NO** |
|---|---|---|
| **TYPE 16** | GCAGTACAAATATGTCATTA | SEQ ID NO: 5 |
| **TYPE 18** | CAATATGATGCTACCAAATTTA | SEQ ID NO: 6 |
| **TYPE 31** | CATTTAAAAGTAGTAATTTTA | SEQ ID NO: 7 |
| **TYPE 33** | CAGTACTAATATGACTTTATG | SEQ ID NO: 8 |
| **TYPE 35** | CTAGTGACAGTACATATAAA | SEQ ID NO: 9 |
| **TYPE 39** | TAGTACCAACTTTACATTATC | SEQ ID NO: 10 |
| **TYPE 45** | CAGTACTAATTTAACATTATGTG | SEQ ID NO: 11 |
| **TYPE 51** | TACAAATTTAACTATTAGCACT | SEQ ID NO: 12 |
| **TYPE 52** | ATATAAAAATGAAAATTTTAAGG | SEQ ID NO: 13 |
| **TYPE 56** | AGTACTAACATGACTATTAGTA | SEQ ID NO: 14 |
| **TYPE 58** | CATATAAAAATGATAATTTTAAGG | SEQ ID NO: 15 |
| **TYPE 59** | CAGCACCAATCTTTCTGTGTGTG | SEQ ID NO: 16 |
| **TYPE 66** | ATATGATGCACGTGAAATCAATC | SEQ ID NO: 17 |
| **TYPE 68** | TTTATGATTCTAATAAATTTAAGGA | SEQ ID NO: 18 |

Similarly, the probe disclosed in (iv) comprises a fluorophore and a sequence, wherein said sequence is complementary to and specific for a sequence of the human housekeeping gene which said multiplex PCR amplifies. In this way, a sequence of a marker of said human housekeeping gene may be detected in the composition subjected to multiplex PCR upon amplification thereof by detection in real-time of the light emitted from the fluorophore of a probe comprising a sequence which is complementary to and specific for said sequence of said marker, when said sequence of said probe binds to said sequence of said marker. Consequently, the method, the use of system and

the use of kit of the present invention also permit DNA from said subject to be detected. Preferably each probe for a given human housekeeping gene comprises a sequence selected from any of the sequences shown in **Table 4.**

**Table 4: Sequence of probes used for detection of each second sequence in multiplex real-time PCR**

| **Target** | **Sequence (5' - 3')** | **SEQ ID NO** |
|---|---|---|
| Beta globin | TGCTTCTGACACAACT | SEQ ID NO: 19 |

Said multiplex real-time PCR therefore:
- amplifies a sequence of a marker of a genotype of the human papillomavirus, irrespective of whether said human papillomavirus is associated with development of cervical, oral or anogenital cancer or not, and detects any sequences, thus amplified, that comprise a sequence of a marker of any of the 16, 18, 31, 33, 35, 39, 45, 51, 52, 56, 58, 59, 66 and 68 genotypes of the human papillomavirus; and
- amplifies a sequence of a marker of a human housekeeping gene, and detects any sequence, thus amplified, that comprise a sequence of a marker of said human housekeeping gene.

Thus, when said subject is infected with a human papillomavirus (and said sample comprises DNA from said subject and said human papillomavirus), said multiplex real-time PCR may amplify (i) and (ii), but if said subject is not infected with a human papillomavirus (and said sample comprises DNA from said subject), said multiplex real-time PCR may only amplify (ii). On the other hand, if said sample comprises DNA from said human papillomavirus but not from said subject, said multiplex real-time PCR will only amplify (i), indicative of an invalid amplification procedure. If said sample does not comprise DNA from said subject or from said human papillomavirus said multiplex real-time PCR will not amplify any DNA. Thus, in the method, uses, system and kit of the present invention, when:
- the fourth fluorophore of the probe binding to said second sequence is not detected and the first or second or third fluorophores of the probes binding to a first sequence are not detected (decision box 102 of **Figure 1****),** step (a) is repeated for said subject ("No" linking decision box 102 with block 100 of **Figure 1****);**
- the fourth fluorophore of the probe binding to said second sequence (i.e. a sequence of a marker of said human housekeeping gene) is detected and at least one of the first or second fluorophores of the probes binding to a first sequence (i.e. a sequence of a marker of the 16 or 18 genotypes of the human papillomavirus, respectively) is detected (decision box 104 of **Figure 1****),** a human papillomavirus genotype associated with development of cervical, oral or anogenital cancer is detected (block 106 of **Figure 1****);**
- the fourth fluorophore of the probe binding to said second sequence is detected and the third fluorophore of the probes of said pool of probes binding to a first sequence *(i.e.* a sequence of a marker of any of the 31, 33, 35, 39, 45, 51, 52, 56, 58, 59, 66 and 68 genotypes of the human papillomavirus) is detected (decision box 108 of **Figure 1****),** a human papillomavirus genotype associated with development of cervical, oral or anogenital cancer is detected and the composition comprising the amplified first sequence and second sequence is selected for subsequent subjection to step (b) when referring to the method of the present invention (block 110 of **Figure 1****),** or to means (b) when referring to the system and kit of the present invention.

In a preferred embodiment of the method, the uses of system and kit of the present invention, said heating is from 60 to 70°C.

In a preferred embodiment of the method, the uses of system and kit of the present invention, said heating is from 60 to 65°C.

In a preferred embodiment of the method, the uses of system and kit of the present invention, said heating is for 1 to 20 minutes.

In a preferred embodiment of the method, the uses of system and kit of the present invention, said heating is for 1 to 10 minutes.

In a preferred embodiment of the method, the uses of system and kit of the present invention, said heating is for 3 to 20 minutes.

In a preferred embodiment of the method, the uses of system and kit of the present invention, said heating is for 1 minute.

In a preferred embodiment of the method, the uses of system and kit of the present invention, said cooling consists of cooling to 37 to 45 °C.

In a preferred embodiment of the method, the uses of system and kit of the present invention, said cooling consists of cooling to 41 °C.

In a preferred embodiment of the method, the uses of system and kit of the present invention, said cooling is for at least 2 minutes.

In a preferred embodiment of the method, the uses of system and kit of the present invention, said cooling is for 2 to 10 minutes.

In a preferred embodiment of the method, the uses of system and kit present invention, said cooling is for 2 to 8 minutes.

In a preferred embodiment of the method, the uses of system and kit of the present invention, said cooling is for 2 to 6 minutes.

In a preferred embodiment of the method, the uses of system and kit of the present invention, said cooling is for 4 minutes.

In a preferred embodiment of the method, the uses of system and kit of the present invention, prior to hybridization according to (b), the composition selected in (a) is heated to 95 °C for 10 minutes.

In a preferred embodiment of the method, the uses of system and kit of the present invention, the composition selected in (a) is subjected to heating to at least 60 °C for at least 10 minutes, prior to cooling and subjecting to hybridization according to step (b). More preferably, said heating is to between 60 and 70 °C for 1 to 20 minutes, even more preferably to between 60 and 65 °C for 3 to 10 minutes.

A preferred embodiment of the method, the uses of system and kit of the present invention is additionally for detecting the absence of said human papillomavirus genotype associated with development of cervical, oral or anogenital cancer in said subject, wherein when the fourth fluorophore of the probe binding to said second sequence is detected and the first or second or third fluorophores of the probes binding to a first sequence are not detected, a human papillomavirus genotype associated with development of cervical, oral or anogenital cancer is not detected..

When the probe binding to said second sequence is not detected and no probe binding to a first sequence is detected, said sample is considered to lack sufficient DNA. It may be that the probe binding to said second sequence is not detected due to the presence of inhibitors in the PCR reaction. In a preferred embodiment of the method, the uses of system and kit of the present invention, when the probe binding to said second sequence is not detected and no probe binding to a first sequence is detected, step (a) is repeated for said subject using a new sample obtained therefrom. Said first, second, third and fourth fluorophores of the respective probes (i.e. fluorescent probes) preferably each emit light in different emission maximum wavelengths. Said first, second, third and fourth fluorophores are more preferably different fluorophores selected from the group consisting of hydroxycoumarin, methoxycoumarin, Alexa fluor 350, DY-415, aminocoumarin, Cy2, FAM, Alexa fluor 488, fluorescein FITC, Alexa fluor 430, JOE [6-JOE, SE (6-carboxy-4',5'-dichloro-2',7'-dimethoxyfluorescein, succinimidyl ester)], VIC (2'-chloro-7'phenyl-1,4-dichloro-6-carboxy-fluorescein) fluorophores, Alexa fluor 532, HEX, Cy3, TRITC, Alexa fluor 546, Alexa fluor 555, R-phycoerythrin (PE), rhodamine Red-X, Tamara, Cy3.5 581, Rox, Alexa fluor 568, Red 613, Texas Red, Alexa fluor 594, Alexa fluor 633, allophycocyanin, Alexa fluor 633, Cy5, Alexa fluor 660, Cy5.5, TruRed, Alexa fluor 680 and Cy7. In one embodiment, one of said first, second, third and fourth fluorophores is a fluorophore selected from the group (blue channel) consisting of hydroxycoumarin, methoxycoumarin, Alexa fluor, aminocoumarin, Cy2 and FAM; another of said first, second, third and fourth fluorophores is a fluorophore selected from the group (green channel) consisting of Alexa fluor 488, fluorescein isothiocyanate (FITC), Alexa fluor 430, JOE, VIC, Alexa fluor 532 and HEX; another of said first, second, third and fourth fluorophores is a fluorophore selected from the group (yellow channel) consisting of Cy3, TRITC, Alexa fluor 546, Alexa fluor 555, R-phycoerythrin (PE), rhodamine Red-X, Tamara, Cy3.5 581, Rox, Alexa fluor 568, Red 613, Texas Red and Alexa fluor 594, and another of said first, second, third and fourth fluorophores is a fluorophore selected from the group (red channel) consisting of Alexa fluor 633, allophycocyanin, Alexa fluor 633, Cy5, Alexa fluor 660, Cy5.5, TruRed, Alexa fluor 680 and Cy7. Even more preferably, said first, second, third and fourth fluorophores are each different fluorophores selected from the group consisting of ROX, Cy5, FAM, HEX, JOE and VIC fluorophores. In an exemplified embodiment, said first fluorophore is ROX, said second fluorophore is Cy5, said third fluorophore is FAM and said fourth fluorophore is HEX or JOE or VIC.

Light emitted from each fluorophore is simultaneously detected (when emitting) by a multiplex real-time PCR platform capable of detecting light at each of the emission maximum wavelengths of said first, second, third and fourth fluorophores. Preferably, said multiplex real-time PCR platform is selected from the group consisting of the QuantStudio^{™} 5 Real-Time PCR System (Applied Biosystems), CFX96^{™} Real-Time PCR Detection System (Bio-Rad) and VitroCycler (Vitro S.A.) platforms in which the method, uses, system and kit of the present invention have been validated. Limit of detection and limit of quantification may be determined as described by Forootan et al. (2017) Methods to determine limit of detection and limit of quantification in quantitative real-time PCR (qPCR), Biomol Detect Quantif. 12: 1-6. For example, a threshold and a baseline can be established for each real-time PCR. The baseline of the real-time PCR reaction may, for example, refer to the signal level during the initial cycles of PCR, such as the mean signal obtained during cycles 3 to 15 of the PCR. The threshold can be used to distinguish relevant amplification signal from the background.

Said (b) in the method of the present invention comprises a step of subjecting the amplified first sequence and second sequence comprised in the composition selected in step (a) to hybridization (block 112 of **Figure 1****),** while said (b) in the uses of system and kit of the present invention comprises means for subjecting the amplified first sequence and second sequence comprised in the composition selected in (a) to hybridization. Thus, the DNA amplified by multiplex real-time PCR which is comprised in the composition in which the fourth fluorophore of the probe binding to said second sequence is detected and the third fluorophore of the probes of said pool of probes binding to a first sequence is detected is that which is subjected to hybridization.

Said hybridization comprises binding of an amplified first sequence and the amplified second sequence comprised in the composition selected in (a) to at least one probe attached to a solid support. Each probe attached to a solid support comprises a sequence complementary to and specific for a sequence of a marker for which a forward primer and a reverse primer complementary to and specific for the locus of said marker were provided in said multiplex real-time PCR in (a).

Accordingly, said hybridization comprises binding/annealing of:
- an amplified first sequence comprised in the composition selected in (a) to at least one sequence complementary to and specific for a sequence of a marker for which a forward primer and a reverse primer complementary to and specific for the locus of said marker were provided in said multiplex real-time PCR in (a), and
- an amplified second sequence comprised in the composition selected in (a) to at least one sequence complementary to and specific for a sequence of a marker for which a forward primer and a reverse primer complementary to and specific for the locus of said marker were provided in said multiplex real-time PCR in (a),
wherein said sequence of a marker is comprised in a probe attached to said solid support.

Said solid support is a three-dimensional porous environment preferably selected from the group consisting of a hybridization membrane, a hybridization sheet and a supported hybridization membrane. More preferably, said solid support is a nylon hybridisation membrane, a charged nylon hybridisation membrane, a nitrocellulose sheet or a supported hybridization membrane comprising nitrocellulose supported on a polymer membrane.

Said solid support comprises probes attached thereto, wherein each probe comprises a sequence complementary to and specific for a sequence of a marker for which a forward primer and a reverse primer complementary to and specific for the locus of said marker were provided in said multiplex real-time PCR in step (a), wherein each probe comprising the same sequence is attached to a specific area of said solid support, and wherein each specific area of said solid support is attached to probes comprising the same sequence. Thus, said solid support comprises multiple, nonoverlapping specific areas, each of which comprises multiple probes comprising the same sequence, and which are different from the sequences of probes attached to other specific areas of said solid support. Each specific area is preferably a circular, square or rectangular area of said solid support, more preferably a circular area (*i.e*. a dot or spot). Said specific areas are preferably arranged in an array, grid or ordered pattern, more preferably a square or rectangular array, even more preferably a square array of circular specific areas.

Each probe attached to a specific area of said solid support comprises a sequence complementary to and specific for a sequence of a marker for which a forward primer and a reverse primer complementary to and specific for the locus of said marker were provided in said multiplex real-time PCR in step (a). Preferably each probe attached to a specific area of said solid support comprises a sequence selected from any of the sequences shown in **Table 5** which is complementary to and specific for a sequence of the corresponding human papillomavirus genotype or human housekeeping gene indicated therein.

**Table 5: Sequence of probes used for detection of each human papillomavirus genotype**

| **Target** | **Sequence (5' - 3')** | SEQ ID NO: |
|---|---|---|
| **TYPE 16** | GAGTACCTACGACATGGG | 20 |
| **TYPE 18** | GCAGTATAGCAGACATGTTG | 21 |
| **TYPE 31** | AGAGTATTTAAGACATGGTGAG | 22 |
| **TYPE 33** | AAAGAATATATAAGACATGTTGAAG | 23 |
| **TYPE 35** | GGAATATTTAAGGCATGGTGAA | 24 |
| **TYPE 39** | GGAATATACCAGGCACGTG | 25 |
| **TYPE 45** | AGTACATATGACCCTACTAAGT | 26 |
| **TYPE 51** | TCCAAGTAACTTTAAGCAATATAT | 27 |
| **TYPE 52** | GGAATACCTTCGTCATGGC | 28 |
| **TYPE 56** | TCAGTACCTTAGACATGTGG | 29 |
| **TYPE 58** | GGAATATGTACGTCATGTTGAA | 30 |
| **TYPE 59** | AGAATATGCCAGACATGTGG | 31 |
| **TYPE 66** | TCAATACCTTCGCCATGTG | 32 |
| **TYPE 68** | GGAATATATTAGGCATGTTGAG | 33 |
| **TYPE 26** | AAACAATTTATAAGACATGGCGAA | 34 |
| **TYPE 53** | AACAGTATGTTAGACATGCAG | 35 |
| **TYPE 73** | AAGGAATATTTAAGACATGCAGAA | 36 |
| **TYPE 82** | GCAGTACATTAGGCATGGG | 37 |
| **TYPE 6** | TATAAAGAGTACATGCGTCAT | 38 |
| **TYPE 11** | GAATACATGCGCCATGTG | 39 |
| **TYPE 40** | GGAATATTTGCGTCATGG | 40 |
| **TYPE 42** | CTAATTTTAAGGAATATTTAAGAC | 41 |
| **TYPE 43** | TTAAGGAATACYTGCGGCAT | 42 |
| **TYPE 44** | CAATACATGCGACATGTTGA | 43 |
| **TYPE 54** | GAGTATATWAGACATGTGGA | 44 |
| **TYPE 55** | AATATAAACAATACATGCGACA | 45 |
| **TYPE 61** | CTTTAGGGAATATTTGCGCCATA | 46 |
| **TYPE 62** | AGGCYACCAACTTTAGGGAAT | 47 |
| **TYPE 67** | AATACCTTAGACATGTGGAA | 48 |
| **TYPE 69** | TTATAAGCAGTTTATAAGGCAT | 49 |
| **TYPE 70** | TATATAGCCCTACAAAGTTTAAGG | 50 |
| **TYPE 71** | TAAAGCCTCTAGTTTCATGG | 51 |
| **TYPE 72** | TATCTTCGCCACACTGAG | 52 |
| **TYPE 81** | AATTTCTGCGCCATACAGA | 53 |
| **TYPE 84** | AATACCTAAGACATGTGGA | 54 |
| **Beta globin** | TGCTTCTGACACAA | 55 |
| **UNIVERSAL HPV PROBE** | MGDCATGYYGARGAAT | 56 |

Any given specific area of said solid support comprises probes, each having a sequence complementary to and specific for a sequence of the same marker. The set of specific areas comprises at least two subsets of specific areas, wherein:
- each specific area of one subset of said specific areas comprises probes, each having a sequence complementary to and specific for a sequence of the same marker for which a forward primer and a reverse primer complementary to and specific for the locus of said marker of a human papillomavirus were provided in said multiplex real-time PCR in step (a); and
- each specific area of one subset of said specific areas comprises probes, each having a sequence complementary to and specific for a sequence of the same marker for which a forward primer and a reverse primer complementary to and specific for the locus of said marker of the human housekeeping gene were provided in said multiplex real-time PCR in step (a),
wherein each probe comprising the same sequence is attached to a specific area of said solid support and each specific area of said solid support is attached to probes comprising the same sequence. When the fourth fluorophore of the probe binding to said second sequence is detected and the third fluorophore of the probes binding to a first sequence is detected in step (a), at least one specific area of said solid support to which a probe is attached may hybridize to a first sequence. Thus, when the fourth fluorophore of the probe binding to said second sequence is detected and the third fluorophore of the probes binding to a first sequence is detected in step (a), if only one genotype of human papillomavirus is infecting said subject and said genotype is identifiable by a marker comprising a sequence complementary to and specific for a sequence of a probe attached to said solid support, wherein said sequence of said probe is complementary to and specific for a sequence of a marker for which a forward primer and a reverse primer complementary to and specific for the locus of said marker were provided in said multiplex real-time PCR in step (b), then said first sequence will hybridize to the sequence of said probe. Preferably, at least one specific area of said solid support to which a probe is attached may hybridize to a first sequence, and at least one specific area of said solid support to which a probe is attached may hybridize to a second sequence.

Said (d) in the method of the present invention comprises a step of identifying each specific area of said solid support to which a probe is attached and to which a first sequence or second sequence is hybridized (block 114 of **Figure 1****),** while said (c) in the uses of system and kit of the present invention comprises means for identifying each specific area of said solid support to which a probe is attached and to which a first sequence or second sequence is hybridized.

Each specific area of said solid support on which hybridization of an amplified sequence and/or an amplified first sequence and second sequence (*i.e.* amplicons) of the composition selected in step (a) to a sequence of a probe attached to said solid support takes place can be detected and hence identified. Detection comprises detection of light emitted from a fluorophore comprised in the probe comprising a sequence to which any given amplicon binds/anneals, as said amplicon binds/anneals thereto, or by use of a molecular tag attached to said amplicons, wherein said molecular tag can be detected. Preferably, detection comprises detection of light (*i.e*. visualisation), wherein said molecular tag is a fluorophore or a reagent necessary for causing a colorimetric reaction. More preferably, and as exemplified, detection comprises visualisation of a molecular tag attached to said amplicons, wherein said tag is biotin which is visualized by an immunoenzymatic colorimetric reaction with streptavidin-phosphatase and a chromogen such as 5-bromo-4- chloro-3-indolyl phosphate (BCIP) and nitro blue tetrazolium (NBT) (also referred to herein as NBT-BCIP herein) and/or with streptavidin-horseradish peroxidase and 3,3 Diaminobenzidine (DAB), generating insoluble precipitates in the solid support in those specific areas in which hybridization has taken place. Light shone through the solid support is scattered to a greater degree in those specific areas in which said insoluble precipitates are present (*i.e.* in which hybridization has taken place), thereby allowing those specific areas in which hybridization has taken place to be identified. Usually, detection includes the detection of concentrations of amplicons compared to grey signal intensities and background noise. A suitable software may define the threshold parameters to distinguish grey intensities and background noise from amplicon signals to perform the analysis of every dot automatically.

The molecular tag may thus be any suitable molecular tag. Suitable molecular tags are known to the skilled person. For example, the molecular tag may be biotin, digoxigenin or a fluorophore as also described elsewhere herein. Biotin may be visualized by an immunoenzymatic colorimetric reaction with streptavidin-phosphatase and a suitable chromogen (e.g. NBT-BCIP) and/or with streptavidin-horseradish peroxidase and 3,3 Diaminobenzidine (DAB). Methods how biotin comprised in DNA/RNA may be visualized on solid supports are *inter alia* described by Leary et al.

(1983) PNAS, Vol. 80, pp. 4045-4049, Genetics. Visualization of digoxigenin-labeled amplicons can be performed in a similar way utilizing anti-digoxigenin antibodies coupled either to alkaline phosphatase (AP) or horseradish peroxidase (HRP) for colorimetric detection. Consequently, the molecular tag such as biotin or digoxigenin can be identified by streptavidin comprising a reporter. The reporter can be any suitable reporter. For example, the reporter may be horseradish peroxidase or alkaline phosphatase.

Therefore, the present invention also is characterised by a use of a forward and/or reverse primer for obtaining an amplified sequence comprising a molecular tag by multiplex real-time PCR, wherein said multiplex real-time PCR amplifies a sequence comprised in DNA present in a biological sample obtained from a subject, wherein said DNA is extracted from said biological sample prior to subjecting it to multiplex real-time PCR,
wherein said forward and/or reverse primer comprises
   - a sequence complementary to and specific for one or more locus of a marker of the genotype 16, 18, 31, 33, 35, 39, 45, 51, 52, 56, 58, 59, 66 and/or 68 of the human papillomavirus; and
   - a molecular tag; and
for identifying a specific area on a solid support to which a probe is attached, wherein said probe comprises a sequence complementary to and specific for a sequence of a marker for which said forward and/or reverse primer is complementary to and specific for; and to which said amplified sequence comprising a molecular tag is hybridized.

A primer (forward and reverse) as described herein is an oligonucleotide. Such oligonucleotide can have a length of 4, 5, 6, 7, 8, 9, 10, 11, 12, 13, 14, 15, 16, 17, 8, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more nucleotides. For example, the oligonucleotide may have a length of 4-50 nucleotides, 5-30 nucleotides, 10-30 nucleotides or 15-30 nucleotides. The primer/oligonucleotide may have a sequence of any one of SEQ ID No. 1-4. The primer as described herein is used for amplification of DNA sequence(s) as described herein.

A primer as described herein may comprise a molecular tag. It is also contemplated that a primer can comprise 1, 2, 3, 4, or more molecular tags. The molecular tag may be attached to a primer by any suitable means. The molecular tag may be attached to the 5' end, the 3' end, or can be attached internally within the primer sequence. Thus, the forward and/or reverse primer may comprise a molecular tag at its 5'-end. Means for attaching a molecular tag to a primer are known to a skilled person and for example described by Benktström et al. (1991) Biotinylation of Oligonucleotides and Their Use as Polymerase Chain Reaction Primers. Nucleosides & Nucleotides 10(1):507-509. Different companies synthesize oligonucleotides comprising a molecular tag on demand such as TriLink^{®} BioTechnologies or Creative Biogene. For example, the molecular tag may be attached to the forward and/or reverse primer, preferably at its 5' end, via a suitable linker. Such suitable linkers are known in the art. For example, the linker may be a 6-(Trifluoroacetylamino)-hexyl-(2-cyanoethyl)-(N,N-diisopropyl)-phosphoramidite (TFA-amino linker), 2-[2-(4-Monomethoxytrityl)aminoethoxy]ethyl-(2-cyanoethyl)-N,N-diisopropyl)-phosphoramidite, 6-(4-Monomethoxytritylamino)hexyl-(2-cyanoethyl)-(N,N-diisopropyl)-phosphoramidite, 6-(4,4'-Dimethoxy-4"-methylsulfonyl-tritylamino)hexyl-(2-cyanoethyl)-(N,N-diisopropyl)-phosphoramidite, 12-(4-Monomethoxytritylamino)dodecyl-1-[(2-cyanoethyl)-(N,N-diisopropyl)]-phosphoramidite, 6-(Trifluoroacetylamino)-hexyl-(2-cyanoethyl)-(N,N-diisopropyl)-phosphoramidite, 10-(O-trifluoroacetamido-N-ethyl)-triethyleneglycol-1-[(2-cyanoethyl)-(N,N-diisopropyl)]-phosphoramidite, 3-(Trifluoroacetylamino)propyl-(2-cyanoethyl)-(N,N-diisopropyl)-phosphoramidite, S-Trityl-6-mercaptohexyl-1-[(2-cyanoethyl)-(N,N-diisopropyl)]-phosphoramidite, 10-Carboxy-decyl-(2-cyanoethyl)-(N,N-diisopropyl)-phosphoramidite, N-hydroxysuccinimide ester, 1-O-Dimethoxytrityl-hexyl-disulfide,1'-[(2-cyanoethyl)-(N,N-diisopropyl)]-phosphoramidite, 6-(N²-methyl-N'-phthalimidyl)-hexyl-1-[(2-cyanoethyl)-(N,N-diisopropyl)]-phosphoramidite.

As disclosed herein the molecular tag may thus be attached to an amplified sequence (also referred to as amplicon herein) obtained by multiplex real-time PCR. The molecular tag can thus be attached to the 5' end of the amplified sequence (e.g. first and/or second sequence) or within the amplified sequence, depending on the position of the molecular tag of the primer that generated the amplified sequence. Preferably, the amplified sequence (e.g. first and/or second amplified sequence) comprises a molecular tag at its 5' end.

Therefore, the present invention also is characterised by a use of an amplified sequence comprising a molecular tag, wherein said amplified sequence has been obtained in multiplex real-time PCR, wherein said multiplex real-time PCR amplifies a sequence comprised in DNA present in a biological sample obtained from a subject, wherein amplification is performed using a forward and/or reverse primer, wherein said forward and/or reverse primer comprises
- a sequence complementary to and specific for one or more locus of a marker of the genotype 16, 18, 31, 33, 35, 39, 45, 51, 52, 56, 58, 59, 66 and/or 68 of the human papillomavirus; and
   - a molecular tag; and
for identifying a specific area on a solid support to which a probe is attached, wherein said probe comprises a sequence complementary to and specific for a sequence of a marker for which said forward and/or reverse primer is complementary to and specific for; and to which said amplified sequence comprising a molecular tag is hybridized.

In addition, by knowing which fluorophores were detected in (a), and the genotype detectable at each specific area of said solid support (*i.e.* which sequence of a marker of a genotype that the probe attached to each specific area of said solid support comprises a sequence complementary to and specific for), having identified each specific area(s) hybridization has taken place in (c), it is subsequently possible to determine the genotype(s) of human papillomavirus infecting said subject. In particular, when:
(I) the fourth fluorophore of the probe binding to said second sequence is detected and the first fluorophore of the probes binding to a first sequence is detected in step (a) (decision box 116 of **Figure 1****),** the human papillomavirus genotype associated with development of cervical, oral or anogenital cancer which is detected is determined to be the 16 genotype of the human papillomavirus (block 118 of **Figure 1****);**
(II) the fourth fluorophore of the probe binding to said second sequence is detected and the second fluorophore of the probes binding to a first sequence is detected in step (a) (decision box 120 of **Figure 1****),** the human papillomavirus genotype associated with development of cervical, oral or anogenital cancer which is detected is determined to be the 18 genotype of the human papillomavirus (block 122 of **Figure 1****);**
(III) the fourth fluorophore of the probe binding to said second sequence is detected and the third fluorophore of the probes binding to a first sequence is detected in step (a) and at least one specific area of said solid support to which a probe is attached and to which a first sequence is hybridized is identified in step (c) (decision box 124 of **Figure 1****),** said genotype is determined (block 130 of **Figure 1****)** by:
   (IIIa) identifying each specific area of said solid support to which a probe is attached and to which a first sequence is hybridized (block 126 of **Figure 1****);**
   (Illb) determining which genotype of the human papillomavirus is identified by a marker comprising a sequence complementary to and specific for a sequence of the probe which is attached to each specific area identified in step (IIIa) (block 128 of **Figure 1****).**

Note that clinical management of a subject, when determined to be infected with the 16 or 18 genotypes of human papillomavirus is generally different from that when determined to be infected with any of the 31, 33, 35, 39, 45, 51, 52, 56, 58, 59, 66 and 68 genotypes of the human papillomavirus. Accordingly, if either or both of the 16 and 18 genotypes of human papillomavirus are exclusively detected [i.e. the fourth fluorophore of the probe binding to said second sequence is detected but the third fluorophore of the probes binding to a first sequence is not detected in step (a)], hybridization as defined in (b) need not be carried out, which in itself saves on reagents for hybridization and minimizes waste. However, in practice the 16 and/or 18 genotypes of human papillomavirus are often, if not always, found in the presence of at least one of the 31, 33, 35, 39, 45, 51, 52, 56, 58, 59, 66 and 68 genotypes of the human papillomavirus. Nevertheless, in the especially preferred embodiment wherein the method, the uses of system and kit of the present invention is for detecting one or more genotypes associated with development of cervical, oral or anogenital cancer and for determining the genotype or genotypes of human papillomavirus associated with development of cervical, oral or anogenital cancer which is or are detected therein, and, optionally, one or more genotypes not associated with development of cervical, oral or anogenital cancer, in a subject infected therewith, wherein each genotype associated with development of cervical, oral or anogenital cancer is selected from the group consisting of the 31, 33, 35, 39, 45, 51, 52, 56, 58, 59, 66 and 68 genotypes of the human papillomavirus, the set of probes defined in (iii) preferably still comprises a probe comprising a first fluorophore and a probe comprising a second fluorophore so as to be able to detect and determine the 16 and 18 genotypes of human papillomavirus in said subject.

However, the method of the present invention is for detecting a human papillomavirus genotype associated with development of cervical, oral or anogenital cancer in a subject and for determining the genotype or genotypes of human papillomavirus infecting said subject when said human papillomavirus genotype associated with development of cervical, oral or anogenital cancer is detected therein, wherein each genotype associated with development of cervical, oral or anogenital cancer is selected from the group consisting of the 31, 33, 35, 39, 45, 51, 52, 56, 58, 59, 66 and 68 genotypes of the human papillomavirus, comprising:
(a) subjecting at least one sequence comprised in DNA present in a biological sample obtained from said subject to multiplex real-time PCR, wherein said DNA is extracted from said biological sample prior to subjecting it to multiplex real-time PCR, wherein said multiplex real-time PCR amplifies:
   (i) at least one first sequence comprised in said DNA, wherein each first sequence is a sequence of a marker of a genotype of the human papillomavirus, wherein amplification of each sequence is performed using a forward primer and a reverse primer, each complementary to and specific for the locus of said marker; and
   (ii) a second sequence comprised in said DNA, wherein said second sequence is a sequence of a marker of a human housekeeping gene, wherein amplification of said second sequence is performed using a forward primer and a reverse primer, each complementary to and specific for the locus of said marker,

in a composition comprising:
   (iii) a pool of probes, wherein each probe of said pool of probes comprises a third fluorophore and a sequence complementary to and specific for a sequence of a marker of a genotype selected from the 31, 33, 35, 39, 45, 51, 52, 56, 58, 59, 66 and 68 genotypes of the human papillomavirus, wherein each the 31, 33, 35, 39, 45, 51, 52, 56, 58, 59, 66 and 68 genotypes of the human papillomavirus has a marker comprising a sequence which is complementary to and specific for at least one sequence of a probe of said pool of probes; and
   (iv) a probe comprising a fourth fluorophore and a sequence, wherein said sequence is complementary to and specific for a sequence of a marker of said human housekeeping gene,
wherein each probe that binds to the sequence of a marker complementary thereto and specific therefor is detected,
wherein when:
   - the fourth fluorophore of the probe binding to said second sequence is not detected and the third fluorophore of the probe binding to a first sequence is not detected, step (a) is repeated for said subject;
   - the fourth fluorophore of the probe binding to said second sequence is detected and the third fluorophore of the probes of said pool of probes binding to a first sequence is detected, a human papillomavirus genotype associated with development of cervical, oral or anogenital cancer is detected and the composition comprising the amplified first sequence and second sequence is selected;
(b) subjecting each amplified first sequence and second sequence of the composition selected in step (a) to hybridization on a solid support comprising probes attached thereto, wherein each probe comprises a sequence complementary to and specific for a sequence of a marker for which a forward primer and a reverse primer complementary to and specific for the locus of said marker were provided in said multiplex real-time PCR in step (a),
wherein each probe comprising the same sequence is attached to a specific area of said solid support and each specific area of said solid support is attached to probes comprising the same sequence; and
(c) identifying each specific area of said solid support to which a probe is attached and to which a first sequence or second sequence is hybridized;
wherein when
(III) the fourth fluorophore of the probe binding to said second sequence is detected and the third fluorophore of the probes binding to a first sequence is detected in step (a) and at least one specific area of said solid support to which a probe is attached and to which a first sequence is hybridized is identified in step (c), said genotype is determined by:
   (IIIa) identifying each specific area of said solid support to which a probe is attached and to which a first sequence is hybridized;
   (IIIb) determining which genotype of the human papillomavirus is identified by a marker comprising a sequence complementary to and specific for a sequence of the probe which is attached to each specific area identified in step (Illa).

Similarly, the use of system and kit are for detecting a human papillomavirus genotype associated with development of cervical, oral or anogenital cancer in a subject and for determining the genotype or genotypes of human papillomavirus infecting said subject when said human papillomavirus genotype associated with development of cervical, oral or anogenital cancer is detected therein, wherein each genotype associated with development of cervical, oral or anogenital cancer is selected from the group consisting of the 31, 33, 35, 39, 45, 51, 52, 56, 58, 59, 66 and 68 genotypes of the human papillomavirus, comprising:
(a) means for subjecting at least one sequence comprised in DNA present in a biological sample obtained from said subject to multiplex real-time PCR, wherein said multiplex real-time PCR amplifies:
   (i) at least one first sequence comprised in said DNA, wherein each first sequence is a sequence of a marker of a genotype of the human papillomavirus, wherein amplification of each sequence is performed using a forward primer and a reverse primer, each complementary to and specific for the locus of said marker; and
   (ii) a second sequence comprised in said DNA, wherein said second sequence is a sequence of a marker of a human housekeeping gene, wherein amplification of said second sequence is performed using a forward primer and a reverse primer, each complementary to and specific for the locus of said marker,

in a composition comprising:
   (iii) a pool of probes, wherein each probe of said pool of probes comprises a third fluorophore and a sequence complementary to and specific for a sequence of a marker of a genotype selected from the 31, 33, 35, 39, 45, 51, 52, 56, 58, 59, 66 and 68 genotypes of the human papillomavirus, wherein each the 31, 33, 35, 39, 45, 51, 52, 56, 58, 59, 66 and 68 genotypes of the human papillomavirus has a marker comprising a sequence which is complementary to and specific for at least one sequence of a probe of said pool of probes; and
   (iv) a probe comprising a fourth fluorophore and a sequence, wherein said sequence is complementary to and specific for a sequence of a marker of said human housekeeping gene,
wherein each probe that binds to the sequence of a marker complementary thereto and specific therefor is detected,
wherein when:
   - the fourth fluorophore of the probe binding to said second sequence is not detected and the third fluorophore of the probe binding to a first sequence is not detected, step (a) is repeated for said subject;
   - the fourth fluorophore of the probe binding to said second sequence is detected and the third fluorophore of the probes of said pool of probes binding to a first sequence is detected, a human papillomavirus genotype associated with development of cervical, oral or anogenital cancer is detected and the composition comprising the amplified first sequence and second sequence is selected;
(b) means for subjecting each amplified first sequence and second sequence of the composition selected in (a) to hybridization on a solid support comprising probes attached thereto, wherein each probe comprises a sequence complementary to and specific for a sequence of a marker for which a forward primer and a reverse primer complementary to and specific for the locus of said marker were provided in said multiplex real-time PCR in (a),
wherein each probe comprising the same sequence is attached to a specific area of said solid support and each specific area of said solid support is attached to probes comprising the same sequence; and
(c) means for identifying each specific area of said solid support to which a probe is attached and to which a first sequence or second sequence is hybridized;
wherein when
(III) the fourth fluorophore of the probe binding to said second sequence is detected and the third fluorophore of the probes binding to a first sequence is detected in (a) and at least one specific area of said solid support to which a probe is attached and to which a first sequence is hybridized is identified in (c), said genotype is determined by:
   (IIIa) identifying each specific area of said solid support to which a probe is attached and to which a first sequence is hybridized;
   (Illb) determining which genotype of the human papillomavirus is identified by a marker comprising a sequence complementary to and specific for a sequence of the probe which is attached to each specific area identified in (Illa).

The present invention also contemplates that the method and/or uses comprise extracting DNA of human papillomavirus. The present invention can also include that the system, and/or kit of the uses comprise step (aa) means for extracting DNA of human papillomavirus present in a biological sample obtained from said subject. Means for extracting DNA can be used before using means for subjecting at least one sequence comprised in DNA present in a biological sample obtained from said subject to multiplex real-time PCR, Step (aa) may thus come before step (a) of the system and kit as described herein. Such means can e.g. include a lysis buffer, a high-concentration salt solution e.g. for precipitating proteins, a solution comprising isopropanol for precipitated DNA from high salt solution and/or a solution for eluting DNA such as e.g. Tris-EDTA or nuclease-free water. Also filters and/or matrices such as silica, cellulose and ion exchange can be comprised by the means.

In another preferred embodiment of the method, the uses of system and kit of the present invention, said DNA is extracted from said biological sample by lysis prior to subjecting it to multiplex real-time PCR, and:
- a first control is also subjected to multiplex real-time PCR, as defined in step (a), when subjecting said at least one sequence comprised in DNA present in a biological sample thereto, wherein said first control is a blank subjected to said extraction, and optionally
- a second control is also subjected to multiplex real-time PCR, as defined in step (a), when subjecting said at least one sequence comprised in DNA present in a biological sample thereto, wherein said second control comprises DNA comprising a first sequence which is a sequence of a marker from a genotype of the human papillomavirus which is selected from the 16, 18, 31, 33, 35, 39, 45, 51, 52, 56, 58, 59, 66 and 68 genotypes of the human papillomavirus; and optionally
- the first sequence and second sequence which were amplified in step (a) and said solid support additionally comprise molecules attached thereto, wherein the molecules attached to said solid support are attached to a specific area of said solid support which is different from other specific areas of said solid support to which a probe is attached, wherein the molecules attached to the first sequence and second sequence which were amplified in step (a) and said solid support have the same chemical structure.

Methods for HPV DNA extraction are known to the person skilled in the art. Respective kits are *inter alia* available from MGI (HPV DNA Extraction Kit, Item No.1000028433). Further, methods for extraction of HPV DNA are, for example, described in Example 1. By extraction of DNA from a biological sample at least 0.1 µg, 0.2 µg, 0.3 µg, 0.4 µg, 0.5 µg, 0.6 µg, 0.7 µg, 0.8 µg, 0.9 µg, 1 µg, 1.5 µg, 2 µg or more of DNA can be obtained. Typically, extracted DNA is comprised in an elution (elution buffer). In the multiplex real-time PCR, DNA can be, for example, added in a concentration of about 100 pg/µL⁻¹ to 100 ng/µl. For example, for multiplex real-time PCR about 1 ng/µL⁻¹ to 4 ng/µL⁻¹ of DNA can be included in the PCR master mix.

In another preferred embodiment of the method, the uses of system and kit of the present invention, each probe is located in at least two specific areas of the solid support. This enables to obtain in one assay more than one result for each probe, thus further improving the sensitivity of the method.

In (b) a hybridization control probe or a hybridization control such as biotin protein (B), an endogenous amplification control probe (C) and/or a degenerated universal probe (U) may be attached to specific areas of the solid support.

In the event that a hybridization control probe (B) is employed, after the development of the solid support (i.e. hybridization) required for identification of the specific areas at which hybridization has taken place, an intense signal (coloration/darkening/lightening) shall appear in the specific area(s) to which said hybridization control probe is attached, thereby serving as a quality control for the hybridization. This signal indicates that the hybridization reagents and development have worked properly. If the signal does not appear, it indicates that an error has occurred during the hybridization process or that a reagent has not been used properly. Preferably, this signal allows software to correctly orientate the solid support (chip) so that subsequent analysis of the hybridization results and, hence, the genotyping, is automated.

An endogenous amplification control probe (C) detects the sequence of the human housekeeping gene amplified (co-amplified with each first sequence) in (a) by multiplex real-time PCR. In the event that an endogenous amplification control probe is employed, after the development of the solid support (i.e. hybridization) required for identification of the specific areas at which hybridization has taken place, a sample where a sequence of the human housekeeping gene has been amplified correctly will hybridize (*i.e*. be able to be visualized and have a positive signal) to the specific areas of the solid support to which the endogenous amplification control probe is attached. This signal shows the quality/quantity of the DNA used in (and, hence, resulting from) the amplification by multiplex real-time PCR. A positive signal shows that the amplification has worked correctly and that the quality and quantity of starting DNA was optimal. The absence of signal for this control means that there has been an error during the amplification, a low quality/amount of DNA used in the amplification or the absence of human DNA in the sample. This last case is possible when the number of human cells present in the sample is under the limit of detection. In addition, if no positive signals are detected for any HPV genotype, the software (e.g. hybriSoft software) will preferably generate an automated message that the sample is a blank, that there is inappropriate or insufficient material, and/or that PCR was inhibited. On the other hand, when the sample tests positive at any of the specific areas of said solid support to which a probe comprising a sequence complementary to and specific for a sequence of a marker of a HPV genotype is attached, but the sample does not test positive at any of the specific areas of said solid support to which the endogenous amplification control probe is attached, said software will preferably generate an automated message that there is insufficient material present in the composition. Under such circumstances, the methodology, reagents and the samples must be checked (for, for example, quality), before validating the hybridization results.

In another preferred embodiment of the method, the uses of wees= system and kit of the present invention, a specific area of said solid support which is different from other specific areas of said solid support to which a probe is attached comprises a pool of probes (*i.e*. a universal probe pool, or a degenerated universal probe, U) attached thereto, wherein each probe of said pool of probes comprises a sequence complementary to and specific for a sequence of the conserved region of the *HPV L1* or *HPV L2* gene, wherein at least two probes in said pool of probes comprise sequences different from one another, wherein when:
- a specific area of said solid support to which said pool of probes is attached and to which a sequence is hybridized is identified;
- a specific area of said solid support to which a probe is attached and to which a second sequence is hybridized is identified; and
- no specific area of said solid support to which a probe is attached and to which a first sequence is hybridized is identified,
the genotype of the human papillomavirus is determined to be different from each genotype identifiable by a marker for which a forward primer and a reverse primer complementary to and specific for the locus of said marker were provided in said multiplex real-time PCR in step (a).

In another preferred embodiment of the method, the uses of system and kit of the present invention, the method is additionally for determining whether said subject is at risk of developing cervical, oral or anogenital cancer, wherein when any one of the 16, 18, 31, 33, 35, 39, 45, 51, 52, 56, 58, 59, 66 and 68 genotypes of the human papillomavirus are determined, said subject is determined to be at risk of developing cervical, oral or anogenital cancer.

In another preferred embodiment of the method, the uses of system and kit of the present invention, the method is additionally for determining whether said subject is at risk of developing benign lesions, wherein when any one of the 6, 11, 26, 40, 42, 43, 44, 53, 54, 55, 61, 62, 67, 69, 70, 71, 72, 73, 81, 82 and 84 genotypes of the human papillomavirus are determined, said subject is determined to be at risk of developing benign lesions. Benign lesions are not cervical, oral or anogenital cancer.

In a preferred embodiment of the uses of system and kit of the present invention:
- the means for subjecting DNA present in said biological sample to multiplex real-time PCR comprise a multiplex real-time PCR instrument and multiplex real-time PCR reagents;
- the means for subjecting each first sequence and second sequence which is amplified in the composition selected in (a) to hybridization on a solid support comprise a solid support comprising probes attached thereto, wherein each probe comprises a sequence complementary to and specific for a sequence of a marker for which a forward primer and a reverse primer complementary to and specific for the locus of said marker were provided in said multiplex real-time PCR in (a), and hybridization reagents;
- the means for identifying each specific area of said solid support to which a probe is attached and to which a sequence is hybridized comprise an image-capture device.

Preferably the multiplex real-time PCR instrument is a platform selected from the group consisting of the QuantStudio^{™} 5 Real-Time PCR System (Applied Biosystems), CFX96^{™} Real-Time PCR Detection System (Bio-Rad) and VitroCycler (Vitro S.A.) platforms, as also described above, while said multiplex real-time PCR reagents are those of a human papillomavirus Master Mix necessary to perform multiplex real-time PCR, more preferably a hot-start polymerase, uracil DNA glycosylase, pairs of forward and reverse primers, each complementary to and specific for the locus of a marker of a genotype of the human papillomavirus or a human housekeeping gene, fluorescent probes, PCR reaction buffer, deoxyuridine triphosphate (dUTP) solution, deoxynucleotide triphosphate (dNTP) solution comprising dATP, dCTP, dTTP and dGTP, and molecularly tagged primers.

Preferably, the means for subjecting each first sequence and second sequence which is amplified in the composition selected in (a) to hybridization on a solid support comprise a solid support, as defined herein, comprising probes attached thereto, wherein each probe comprises a sequence complementary to and specific for a sequence of a marker for which a forward primer and a reverse primer complementary to and specific for the locus of said marker were provided in said multiplex real-time PCR in (a), as defined herein, and hybridization reagents. More preferably, said reagents comprise hybridization solution (Reagent A), blocking solution (Reagent B), streptavidine-phosphatase (streptavidine-alkaline phosphatase, Reagent C), washing buffer I (Reagent D), NBT-BCIP solution (Reagent E) and washing buffer II (Reagent F). Even more preferably, the means for subjecting each first sequence and second sequence which is amplified in the composition selected in (a) to hybridization on a solid support also comprise a hybridization platform such as the exemplified hybriSpot 12 or 24 platforms, a thermocycler, a thermal block or thermostatic bath/heater, and a cold plate.

Preferably, the means for identifying each specific area of said solid support to which a probe is attached and to which a sequence is hybridized comprise an image-capture device. Said image capture device is preferably comprised in the aforementioned hybridization platform such as the exemplified hybriSpot 12 or 24 platforms.

Said multiplex real-time PCR instrument, hybridization platform and image-capture device may each comprise a computer program product.

The computer program product may include a computer readable storage medium (or media) having computer readable program instructions thereon for causing a processor to carry out aspects or steps of the present invention.

The computer readable storage medium can be a tangible device that can retain and store instructions for use by an instruction execution device. The computer readable storage medium may include, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention which is defined in the appended claims, may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects or steps of the present invention.

Aspects or steps of the present invention are described herein with reference to flowchart illustrations and/or block diagrams of methods, apparatus (systems), uses and kits according to embodiments and/or steps of the invention (see e.g. Figures 1 and 4). It will be understood that each square or diamond-shaped block of the flowchart illustrations and/or block diagrams, and combinations of blocks in the flowchart illustrations and/or block diagrams, can be implemented by biological techniques or computer readable program instructions, or combinations thereof.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and kits according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

The corresponding structures, materials, acts, and equivalents of all means or step plus function elements in the claims below are intended to include any structure, material, or act for performing the function in combination with other claimed elements as specifically claimed. The description of the present invention has been presented for purposes of illustration and description but is not intended to be exhaustive or limited to embodiments of the invention in the form disclosed. Many modifications and variations will be apparent to those of ordinary skill in the art without departing from the scope of embodiments of the invention which is defined in the appended claims. The embodiment was chosen and described in order to best explain the principles of embodiments of the invention and the practical application, and to enable others of ordinary skill in the art to understand embodiments of the invention for various embodiments with various modifications as are suited to the particular use contemplated. Nonetheless, the invention is defined in the appended claims, only.

### Examples

The following examples illustrate the invention and should not be considered as limiting, but rather illustrative of the invention which is defined in the appended claims.

The following examples are based on the amplification of a fragment in the viral region L1 of papillomavirus by PCR, followed by hybridization onto a membrane with DNA-specific probes by using technology suitable for analysis in both automatic and manual hybriSpot platforms.

**Example 1:** Multiplex real-time PCR of samples obtained from subjects infected with human papillomavirus.

Example 1 is a particular embodiment of (a) of the method, uses, system and kit of the present invention.

### Reagents:

Samples obtained from 24 subjects by use of a swab (self-swab) of the anogenital region, said subjects being infected with human papillomavirus (HPV).

DNase/RNase-free double distilled water was used for the handling of clinical samples:
A lyophilized multiplex real-time PCR mix was prepared for analysis of the 24 clinical samples on a hybriSpot 24 platform. The lyophilized multiplex real-time PCR mix contains PCR buffer, dNTPs (U/T), DNase/RNase-free water, biotinylated primers, DNA polymerase and Uracil-DNA glycosylase (Cod-UNG), and fluorescent probes. The biotinylated primers include one pair of primers which detects at least 35 HPV genotypes, wherein each of the primers is specific for the amplification of a fragment of the HPV L1 region. Furthermore, the remaining biotinylated primers include a pair of primers for the amplification of a human housekeeping gene (beta-globin gene) and used as an internal control for the multiplex real-time PCR. The fluorescent probes used were ROX (for HPV 16 detection), Cy5 (for HPV 18 detection), FAM (for detection of HPV 31, 33, 35, 39, 45, 51, 52, 56, 58, 59, 66 and 68 = HPV HR genotypes) and JOE (for use as an internal control).

A sequence of a marker from a genotype of the human papillomavirus which is selected from the 16, 18, 31, 33, 35, 39, 45, 51, 52, 56, 58, 59, 66 and 68 genotypes of the human papillomavirus was used as a positive control (PC) and DNase/RNase-free DEPC-treated water was used as a negative control (NTC).

Shipment and storage of said reagents was conducted at 2 - 8 °C in the dark, in areas not contaminated with DNA or PCR products.

### Methodology:

Purified genetic material from different types of biological samples, such as liquid-based cytologies and vaginal and rectal swabs has been used. DNA was extracted using DNA/RNA purification kits and extraction equipments* starting with 200 µl of biological sample and eluting in 100 µl of elution buffer (for purification with Opentrons it started with 92 µl of clinical specimen and elute in 60 µl of elution solution):

| **Extraction Kit** | **Extraction Equipment** |
|---|---|
| Maxwell^{®} 16 FFPE Tissue LEV AND Purification Kit (Promega) | Maxwell^{®} 16 (Promega) |
| NX48S - Urine/Swab DNA Kit (Genolution) | Nextractor NX-48S (Genolution) |
| RNA/DNA pathogen extraction kit (Robot Opentrons OT2) (Vitro, ref. MAD-003955M) | Opentrons OT-2 |

Alternatively, DNA was extracted using RNA/DNA Pathogen Extraction kit (for Robot Nextractor^{®} NX-48S) Ref. MAD-003955M-EX.

8 µL of the eluted DNA is added to 12 µL of PCR master mix. Thus, the PCR reaction was carried out in a final volume of 20 µL in each tube containing the multiplex real-time PCR reaction mix to obtain template DNA for the PCR reaction. A a negative control is included by adding 8 µl of the water to 12 µL of PCR master mix. Additionally, a positive control is included by adding 8 µl of the positive DNA control HPV Screening to 12 µL of PCR master mix.

Each tube was placed in the thermocycler and the following steps of PCR amplification shown in **Table 6** were used:

**Table 6: Steps of PCR amplification used**

| **PCR PROGRAM** | | |
|---|---|---|
| 25°C | 5 min | 1 cycle |
| 95°C | 5 min | 1 cycle |
| 95°C | 15 sec | 5 cycles |
| 42°C | 15 sec | |
| 72°C | 30 sec | |
| 95°C | 15 sec | 45 cycles |
| 60°C* | 40 sec | |

| | | |
|---|---|---|
| * Fluorescence data should be collected during the extension stage (*) through ROX (HPV 16), Cy5 (HPV 18), FAM (HPV 31, 33, 35, 39, 45, 51, 52, 56, 58, 59, 66 and 68) and HEX, JOE or VIC (Internal Control) channels. | | |

The following **Table 7** was used to interpret the fluorescence data obtained for the positive and negative controls (PC and NTC, respectively), whereby detection of a signal (the amplification signal) was determined by a rapid and steady increase in the fluorescence data values and not by peak phenomena or gradual increase of the background signal (irregular background or increased background noise) **(****Figure 2A** and **2B****).**

**Table 7: Interpretation of multiplex real-time PCR fluorescence data obtained for the positive (PC) and negative (NTC) controls**

| **Control** | **Data** | **Interpretation** |
|---|---|---|
| PC | Signal for FAM, ROX, Cy5 and JOE channels detected* | The control/reaction is correct |
| | No signal for FAM and/or ROX and/or Cy5 and/or JOE channels detected | Problem with amplification and/or sampling: repeat analysis |
| NTC | Signal for FAM and/or ROX and/or Cy5 and/or JOE channels detected | Contamination: repeat analysis and/or sampling |
| | No signal detected | The control/reaction is correct |

| | | |
|---|---|---|
| *The amplification signal must be determined by a rapid and steady increase in the fluorescence values and not by peak phenomena or gradual increase of the background signal (irregular background or increased background noise) | | |

The PCR run was considered valid when the aforementioned criteria for all reaction controls and the detection count (Ct) values obtained in the positive control for the different targets are within the range of expected values, of 20±2 for HPV 16 (ROX), 20±2 for HPV 18 (Cy5), 20±2 for HPV HR (FAM) and 20±2 for Beta globin (JOE).

The results obtained for each of the clinical samples were interpreted according to the following **Table 8:**

**Table 8: Interpretation of multiplex real-time PCR fluorescence data obtained for each clinical sample**

| **Target (probe)** | | | | **Interpretation** |
|---|---|---|---|---|
| **HPV 16 (ROX)** | **HPV 18 (Cy5)** | **HPV HR (FAM)** | **Beta globin (JOE)** | |
| Signal | No signal | No signal | Signal | Positive for HPV 16 |
| | | | No signal | |
| No signal | Signal | No signal | Signal | Positive for HPV 18 |
| | | | No signal | |
| No signal | No signal | Signal | Signal | Positive for HPV 31, 33, 35, 39, 45, 51, 52, 56, 58, 59, 66 and/or 68 |
| | | | No signal | |
| Signal | Signal | No signal | Signal | Positive for HPV 16 and 18 |
| | | | No signal | |
| Signal | No signal | Signal | Signal | Positive for HPV 16 and any of the HPV 31, 33, 35, 39, 45, 51, 52, 56, 58, 59, 66 and/or 68 |
| | | | No signal | |
| No signal | Signal | Signal | Signal | Positive for HPV 18 and any of the HPV 31, 33, 35, 39, 45, 51, 52, 56, 58, 59, 66 and/or 68 |
| | | | No signal | |
| Signal | Signal | Signal | Signal | Positive for HPV 16 and HPV 18 and any of HPV 31, 33, 35, 39, 45, 51, 52, 56, 58, 59, 66 and/or 68 |
| | | | No signal | |
| No signal | No signal | No signal | Signal | Negative result* |
| | | | No signal | Invalid result^{‡} |

| | | | | |
|---|---|---|---|---|
| * Negative or below the limit of detection. ‡ Problem with extraction and/or amplification | | | | |

The default threshold line was established automatically by the VitroCycler (Vitro S.A.) instrument or manually adjusted, where necessary, until it lies within the exponential phase of the fluorescence curves and above any background signal.

A sample was considered positive if the Ct value obtained was ≤ 40, even if the internal control did not show amplification (sometimes the internal control was not amplified correctly due to the presence of a high initial number of copies of target bacterial nucleic acid, which can cause a preferential amplification of the latter).

A sample was considered negative if amplification was not detected over the threshold value, and if the internal control did show it. The inhibition of the PCR reaction can be excluded by the amplification of the internal control.

It was established that the method of the present invention has a limit of detection of 10 copies/reaction for the HPV 16, HPV 18, HPV 31, HPV 33, HPV 35, HPV 39, HPV 45, HPV 51, HPV 52, HPV 56, HPV 58, HPV 59, HPV 66 and HPV 68 genotypes.

**Example 2:** Hybridization of amplicons from Example 1.

Example 2 is a particular embodiment (c) of the method, uses, system and kit of the present invention.

### Reagents:

The biotinylated amplicons generated during PCR from Example 1 were used for hybridization.

The following reagents were prepared for analysis of the 24 clinical samples obtained from subjects infected with human papillomavirus (HPV) on a hybriSpot 24 platform. Said reagents comprise hybridization solution (Reagent A), blocking solution (Reagent B), streptavidine-phosphatase (streptavidine-alkaline phosphatase, Reagent C), washing buffer I (Reagent D), Substrate+ Chromogen (Reagent E) and washing buffer II (Reagent F). Reagent A was pre-heated to 41 °C in a thermostatic bath or heater immediately prior to use), while the remaining Reagents B to F were used at room temperature (20 - 25 °C).

The solid support used was a hybriSpot chip (membrane) comprising probes attached to specific areas thereof. Each chip comprised four subsets of specific areas, wherein:
- each specific area of one subset of said specific areas comprised probes, each probe having a sequence selected from SEQ ID NO: 20 to SEQ ID NO: 54 complementary to and specific for a sequence of a marker of a human papillomavirus, wherein each probe comprising the same sequence is attached to a specific area of said solid support;
- each specific area of one subset of said specific areas comprised probes having a sequence of SEQ ID NO: 55 complementary to and specific for a sequence of a marker of the human *HBB* gene (endogenous amplification control, C), wherein each probe comprising the same sequence is attached to a specific area of said solid support.
- the biotin protein served as hybridization control.
- each specific area of one subset of said specific areas comprised a degenerated universal probe having a sequence selected from SEQ ID NO: 56.

Shipment and storage of said reagents was conducted at 2 - 8 °C in areas not contaminated with DNA or PCR products.

### Methodology:

The biotinylated amplicons generated during PCR in Example 1 were hybridized onto membranes containing an array of specific probes for each HPV genotype associated with development of cervical, oral and/or anogenital cancer, as well as amplification and hybridization control probes. The hybridization process was performed semi-automatically on the hybriSpot (HS12) platform following the instructions provided by the wizard of said platform. Specifically, the reagents are added manually and the incubation times, temperature and draining of reagents are controlled by a firmware. However, the hybridization was also fully automated on the hybriSpot HS12a or HS24 platforms in parallel, achieving the same results (not shown here).

The technology allowed very fast binding of the PCR product and its specific probe in the three-dimensional porous environment of the membrane, as compared to the hybridization on a conventional surface. Once binding between the specific amplicons and their corresponding probes had occurred, the signal was visualized by an immunoenzymatic colorimetric reaction with Streptavidin-Phosphatase and a chromogen (NBT-BCIP) generating insoluble precipitates in the membrane in those specific areas thereof in which hybridization took place.

The management of the samples, the capture of images and the analysis and report of the results were performed automatically by the hybriSoft^{™} software.

Before starting the hybridization process, the following steps were carried out:
(i) denaturing the biotinylated amplicons generated during PCR in Example 1 by heating at 95 °C for 10 minutes in a thermocycler and cool quickly in ice for at least 2 minutes;
(ii) preheating Reagent A to 41 °C; and
(iii) placing each HPV Chip (one per valid clinical sample which tested positive for HPV 31, 33, 35, 39, 45, 51, 52, 56, 58, 59, 66 and/or 68 in Example 1) in the position indicated in the platform (HS12).

Notably, the PCR product added to the array contained the remains of Taqman probes from the Real Time PCR that have not been exhausted during amplification, so there will be two complementary probes in the hybridization device, one in liquid phase and the other in solid phase, and both could compete to hybridize with the amplicon of interest. To prevent this from happening, and to ensure that the generated amplicon hybridizes completely with the solid-phase probe, i.e. the one in the array, we performed a first hybridization step using a switch in temperature. The temperature is first set at 60°C. With this the Taqman probe remnants separate from the liquid phase amplicon and avoid their hybridization, improving the sensitivity of the technique. This happens because the temperature is above the melting temperature of all the probes, both those of the array and those of the Real Time. If that initial temperature rise does not occur, some of the Taqman probe debris could hybridize to the amplicon of interest and prevent it from binding to the chip probe.

Once all the samples have been added to the arrays, they have been incubated 1 minute at 60°C and subsequently the temperature was lowered to 41°C. Specifically, the samples were incubated for an additional 6 minutes at 41°C.

By setting the temperature at 41°C, the PCR products (amplicons, amplified DNA) bind to the array probes according to the melting temperatures of these probes.

After this incubation at 41°C for 6 minutes, the hybridization protocol continues as described below.

The manual hybridization protocol comprises the following steps:
(a) setting the temperature of the equipment at 60 °C and adding 300 µL of Reagent A (Hybridization Solution) preheated for at 41 °C to each chip and incubating until temperature reaches 60 °C;
(b) once the temperature has reached 60 °C removing Reagent A (Hybridization Solution) by activating the vacuum pump;
(c) mixing 20 µL of each PCR sample (previously denatured and kept on ice) with 270 µL of Reagent A (Hybridization Solution at 41 °C), and dispensing the mixture onto the corresponding HVP Chip
(d) incubating for 1 min at 60 °C
(e) setting the temperature of the equipment at 41°C and once it is reached incubating for 6 min.
(f) activating the pump for at least 30 seconds to remove the liquid containing the PCR products (comprising amplicons, amplified DNA as obtained by multiplex real-time PCR);
(g) washing 3 times with 300 µL of Reagent A (Hybridization Solution at 41 °C);
(h) setting the temperature to 29 °C;
(i) adding 300 µL of Reagent B (Blocking Solution) and incubating for 5 minutes;
(j) activating the pump to remove Reagent B;
(k) adding 300 µL of Reagent C (Streptavidin-Alkaline Phosphatase) to each chip once the temperature has reached 29 °C;
(l) incubating for 5 minutes to 29 °C;
(m) activating the pump to remove the reagent;
(n) setting the temperature to 36 °C;
(o) washing the membranes 4 times with 300 µL with Reagent D (Washing buffer I);
(p) adding 300 µL of Reagent E to each chip once the temperature has reached 36 °C, and incubating for 10 minutes at 36 °C;.
(q) activating the pump to remove the reagent E that contains the substrate and chromogen to perform the enzymatic colorimetric reaction;
(r) washing the membranes 2 times with 300 µL of Reagent F (Washing buffer II).
(s) activating the pump to remove the reagent F (buffer with very low salt concentrations) stopping enzymatic colorimetric reaction; and
(t) performing image capture, analysis and reporting of results following the instructions of the HS12 user manual.

**Example 3:** Determination of the 56 genotype of said human papillomavirus infecting said subject.

Example 3 is a particular embodiment of (c) of the method, uses, system and kit of the present invention. It was carried out for one of the 24 samples obtained from subjects which, after subjection to multiplex real-time PCR according to Example 1, tested positive for HPV 31, 33, 35, 39, 45, 51, 52, 56, 58, 59, 66 and/or 68 as per the third row of Table 8. Subsequent subjection to hybridisation on a solid support according to Example 2 resulted in hybridisation being visualized as having taken place at specific areas denoted by:
(i) specific areas 1,A; 1,B; 2,I; 5,E and 8,A of **Figure 3B** (specific areas are identified by a numeral and letter of the alphabet, respectively, counted from the top left specific area 1A of said solid support) which correspond to those specific areas to which the hybridization control probe (B) was attached according to the schematic of **Figure 3C****;**
(ii) specific areas 1,C and 5,F of **Figure 3B** which correspond to those specific areas to which the endogenous amplification control probe (C) was attached according to the schematic of **Figure 3C****;** and
(iii) specific areas 1,D and 5,G of **Figure 3B** which correspond to those specific areas to which the HPV degenerated universal probe (U) was attached according to the schematic of **Figure 3C****,** thereby indicating that hybridization was valid. In addition, hybridisation was visualized as having taken place at specific areas denoted by:
(iv) specific areas 2,H and 7,C of **Figure 3B** which correspond to those specific areas to which the probe of the HPV 56 genotype was attached according to the schematic of **Figure 3C** the degenerated HPV universal probe (U) were attached.

Accordingly, the subject from whom said sample was obtained was determined to be infected with the HPV 56 genotype which is associated with development of cervical, oral or anogenital cancer in a subject.

**Example 4:** Comparison of different hybridization conditions.

The same group of samples (liquid cytology samples with previous positive results for HR HPV genotypes by real-time PCR) was assayed with different assay conditions. The results are shown in Figure 5.

Twelve clinical samples that had a previous positive result for HPV by real-time PCR screening without hybridization were used. The previous real-time PCR results, without hybridization, are shown in Figure 5.

An extraction of genetic material from the samples was performed with the RNA/DNA Pathogen Extraction assay (VITRO SA, Granada, Spain) and a total volume of 60 µl of sample was obtained. With these samples three hybridization conditions were tested:
Hybridization conditions 1: Real-time PCR, followed by hybridization of the PCR product at a temperature of 41°C. 8 µl of each sample were mixed with 12 µl of a Master Mix necessary to perform multiplex real-time PCR with the Vitro HPV Screening assay. The products of this real-time PCR were denatured by heating at 95 °C during 10 min and hybridized onto a membrane with HPV-specific probes using DNAFlow technology on a hybriSpot platform at a temperature of 41°C.
Hybridization conditions 2: Amplification of the samples by an end point PCR without fluorescent probes, followed by hybridization of the PCR product at a temperature of 41°C. This is considered the gold standard method. 30 µl of each sample were subjected to a PCR amplification with a mix (lyophilized PCR mix) that didn't contain any fluorescent probe. No interference with the probes on the solid surface should occur under these conditions. The products of this real-time PCR were denatured by heating at 95 °C during 10 min and hybridized onto a membrane with HPV-specific probes using DNAFlow technology on a hybriSpot platform at a temperature of 41°C.
Hybridization conditions 3: Real-time PCR, followed by hybridization of the PCR product at a temperature of 60 °C, and subsequent cooling to 41 °C, according to the method of the invention. 8 µl of each sample were mixed with 12 µl of a Master Mix necessary to perform multiplex real-time PCR with the Vitro HPV Screening assay. The products of this real-time PCR were denatured by heating at 95 °C during 10 min and hybridized onto a membrane with HPV-specific probes using DNAFlow technology on a hybriSpot platform with a temperature gradient starting at 60°C and ending at 41°C. The products remain at 60°C for 1 minute, then the temperature started gradually to decrease to 41°C during 4 minutes, and finally the products remained at 41°C for 6 minutes.

For all the conditions the whole hybridization process is performed automatically and the management of the samples, the capture of images and the analysis and report of the results are performed through the hybriSoft^{™} software. The same lot of reagents were used for all the conditions.

When the real-time PCR is followed by hybridization of the PCR product at a temperature of 41°C (not according to the invention), some genotypes are not identified (see hybridization conditions 1 in Figure 5): genotype 18 is not identified in sample M1, genotype 16 is not identified in sample M2, genotypes 18, 53 and 67 are not identified in sample M4, genotypes 16 and 58 are not identified in sample M5, genotype 16 is not identified in sample M6, genotype 16 is not identified in sample M7, genotype 16 is not identified in sample M8, genotype 18 is not identified in sample M9, and genotype 18 is not identified in sample M11. All these genotypes are properly identified when the real-time PCR is followed by hybridization of the PCR product at a temperature of 60 °C, and subsequent cooling to 41 °C, according to the method of the invention (see hybridization conditions 3 in Figure 5), as well as in the gold standard method (see hybridization conditions 2 in Figure 5).

The PCR product added to the array contained the probes remaining from the real-time PCR that have not been exhausted during amplification, so there will be two complementary probes in the hybridization device, one in liquid phase and the other in solid phase, and both could compete to hybridize with the amplicon of interest. This is prevented by hybridization of the PCR product at a temperature of 60 °C, which promotes that the generated amplicon hybridizes preferentially with the solid-phase probe, i.e. the one in the array, and not with the probes remaining from the PCR in the liquid phase, improving the sensitivity and specificity of the method. This happens because the temperature is above the melting temperature of all the probes, both those of the array and those of the real-time PCR. If the heating temperature is below 60 °C, some of the probes remaining from the PCR could hybridize to the amplicon of interest and prevent it from binding to the chip probe.

This example demonstrates that when the real-time PCR is followed by hybridization of the PCR product at a temperature of 60 °C, and subsequent cooling to 41 °C, according to the method of the invention, the genotypes present in the sample are properly detected and identified. When the real-time PCR is followed by hybridization of the PCR product at a temperature of 41°C, some genotypes are not identified.

## Claims

1. A method for detecting and determining a human papillomavirus genotype associated with development of cervical, oral or anogenital cancer in a subject, wherein said human papillomavirus genotype is selected from the group consisting of: 31, 33, 35, 39, 45, 51, 52, 56, 58, 59, 66, and 68, the method comprising:
(a) subjecting at least one sequence comprised in DNA present in a biological sample that has been obtained from said subject to multiplex real-time PCR, wherein said DNA is extracted from said biological sample prior to subjecting it to multiplex real-time PCR that amplifies:
(i) at least one first sequence of a marker of a genotype of the human papillomavirus, wherein amplification of said first sequence is performed using a forward primer and a reverse primer, specific for the locus of said marker; and
(ii) a second sequence of a marker of a human housekeeping gene, wherein amplification of said second sequence is performed using a forward primer and a reverse primer, specific for the locus of said marker,
in a composition comprising:
(iii) a set of probes comprising a pool of probes, wherein each probe of said pool of probes comprises a fluorophore combined with a sequence specific for a sequence of a marker of a human papillomavirus genotype selected from the group consisting of: 31, 33, 35, 39, 45, 51, 52, 56, 58, 59, 66, and 68, wherein each human papillomavirus genotype has a marker comprising a sequence which is specific for at least one sequence of a probe of said pool of probes; and
(iv) a probe comprising a fluorophore combined with a sequence specific for said second sequence,
wherein each probe that binds to the sequence of a marker specific therefor is detected,
wherein when:
- the fluorophore of the probe binding to said second sequence is not detected and the fluorophore of said pool of probes binding to said first sequence is not detected, step (a) is repeated for said subject;
- the fluorophore of the probe binding to said second sequence is detected and the fluorophore of said pool of probes binding to said first sequence is detected, a human papillomavirus genotype associated with development of cervical, oral or anogenital cancer is detected and the composition comprising the amplified first sequence and second sequence is selected;
(b) subjecting each amplified first sequence and second sequence of the composition selected in step (a) to hybridization on a solid support comprising probes attached thereto, wherein during hybridization, the composition selected in step (a) is subjected to heating to at least 60 °C and subsequently, to gradual cooling, wherein, under said hybridization conditions, the amplicons generated in previous step (a) hybridize preferentially to the probes in solid phase, which are attached to the solid support, and not to the probes in the liquid phase, which remain from the real-time multiplex PCR of step (a), wherein each probe comprises a sequence specific for a sequence of a marker for which a forward primer and a reverse primer specific for the locus of said marker were provided in said multiplex real-time PCR in step (a),
wherein each probe comprising the same sequence is attached to a specific area of said solid support and each specific area of said solid support is attached to probes comprising the same sequence; and
(c) identifying each specific area of said solid support to which a probe is attached and to which a first sequence or second sequence is hybridized;
wherein when the fluorophore of the probe binding to said second sequence is detected and the fluorophore of said pool of probes binding to said first sequence is detected in step (a) and at least one specific area of said solid support to which a probe is attached and to which a first sequence is hybridized is identified in step (c), said genotype is determined by:
(la) identifying each specific area of said solid support to which a probe is attached and to which a first sequence is hybridized;
(lb) determining which genotype of the human papillomavirus is identified by a marker comprising a sequence specific for a sequence of the probe which is attached to each specific area identified in step (la).

2. Use of a system for detecting and determining a human papillomavirus genotype associated with development of cervical, oral or anogenital cancer in a subject, wherein said human papillomavirus genotype is selected from the group consisting of: 31, 33, 35, 39, 45, 51, 52, 56, 58, 59, 66, and 68, the system comprising:
(a) means for subjecting at least one sequence comprised in DNA present in a biological sample obtained from said subject to multiplex real-time PCR that amplifies at least one first sequence of a marker of a genotype of the human papillomavirus of claim 1 and the second sequence of a marker of a human housekeeping gene of claim 1,
in a composition comprising the pool of probes of claim 1 and the probe comprising a fluorophore combined with a sequence specific for said second sequence of claim 1,
wherein each probe that binds to the sequence of a marker specific therefor is detected,
wherein when
- the fluorophore of the probe binding to said second sequence is detected and the fluorophore of said pool of probes binding to said first sequence is detected, a human papillomavirus genotype associated with development of cervical, oral or anogenital cancer is detected and the composition comprising the amplified first sequence and second sequence is selected, and
wherein the means for subjecting at least one sequence to multiplex real-time PCR comprise: a multiplex real-time PCR instrument; the forward primer and the reverse primer, specific for the locus of the at least one said first sequence; the forward primer and the reverse primer, specific for the locus of the sequence specific for said second sequence; the pool of probes, wherein each probe of said pool of probes comprises a fluorophore combined with a sequence specific for a sequence of a marker of a human papillomavirus genotype of claim 1; and the probe comprising the fluorophore combined with the sequence specific for said second sequence; and multiplex real-time PCR reagents;
(b) means for subjecting each amplified first sequence and second sequence of the composition selected in (a), to hybridization on a solid support comprising probes attached thereto, means for heating the composition selected in (a) to at least 60 °C during hybridization, and means for cooling the composition selected in (a) during hybridization, wherein each probe comprises a sequence specific for a sequence of a marker for which a forward primer and a reverse primer specific for the locus of said marker were provided in said multiplex real-time PCR in (a),
wherein each probe comprising the same sequence is attached to a specific area of said solid support and each specific area of said solid support is attached to probes comprising the same sequence,
wherein the means for heating the composition selected in (a) comprise a heating device,
wherein the means for cooling the composition selected in (a) comprise a cooling device,
wherein the means for subjecting each first sequence and second sequence which is amplified in the composition selected in (a) to hybridization on a solid support comprise a solid support comprising probes attached thereto, and hybridization reagents, wherein said solid support comprises said pool of probes, wherein each probe of said pool of probes comprises a fluorophore combined with a sequence specific for a sequence of a marker of said human papillomavirus genotype of claim 1; and the probe comprising the fluorophore combined with the sequence specific for said second sequence; and
(c) means for identifying each specific area of said solid support to which a probe is attached and to which a first sequence or second sequence is hybridized, wherein said means comprise an image-capture device;
wherein when the fluorophore of the probe binding to said second sequence is detected and the fluorophore of said pool of probes binding to said first sequence is detected in (a) and at least one specific area of said solid support to which a probe is attached and to which a first sequence is hybridized is identified in (c), said genotype is determined by:
(la) identifying each specific area of said solid support to which a probe is attached and to which a first sequence is hybridized;
(lb) determining which genotype of the human papillomavirus is identified by a marker comprising a sequence specific for a sequence of the probe which is attached to each specific area identified in (la); and
wherein said use comprises subjecting each amplified first sequence and second sequence of the composition selected in (a), to hybridization on a solid support comprising probes attached thereto, wherein during hybridization, the composition selected in (a) is subjected to heating to at least 60 °C and subsequently, to cooling, and wherein, under said hybridization conditions, the amplicons generated in the real-time multiplex PCR hybridize preferentially to the probes in solid phase, which are attached to the solid support, and not to the probes in the liquid phase, which remain from the real-time multiplex PCR.

3. Use of a kit for detecting and determining a human papillomavirus genotype associated with development of cervical, oral or anogenital cancer in a subject, wherein said human papillomavirus genotype is selected from the group consisting of: 31, 33, 35, 39, 45, 51, 52, 56, 58, 59, 66, and 68, the kit comprising:
(a) means for subjecting at least one sequence comprised in DNA present in a biological sample obtained from said subject to multiplex real-time PCR that amplifies at least one first sequence of a marker of a genotype of the human papillomavirus of claim 1 and the second sequence of a marker of a human housekeeping gene of claim 1,
in a composition comprising the pool of probes of claim 1 and the probe comprising a fluorophore combined with a sequence specific for said second sequence of claim 1,
wherein each probe that binds to the sequence of a marker specific therefor is detected,
wherein when
- the fluorophore of the probe binding to said second sequence is detected and the fluorophore of said pool of probes binding to said first sequence is detected, a human papillomavirus genotype associated with development of cervical, oral or anogenital cancer is detected and the composition comprising the amplified first sequence and second sequence is selected, and
wherein the means for subjecting at least one sequence to multiplex real-time PCR comprise: a multiplex real-time PCR instrument; the forward primer and the reverse primer, specific for the locus of the at least one said first sequence; the forward primer and the reverse primer, specific for the locus of the sequence specific for said second sequence; the pool of probes, wherein each probe of said pool of probes comprises a fluorophore combined with a sequence specific for a sequence of a marker of a human papillomavirus genotype of claim 1, and the probe comprising the fluorophore combined with the sequence specific for said second sequence; and multiplex real-time PCR reagents;
(b) means for subjecting each amplified first sequence and second sequence of the composition selected in (a), to hybridization on a solid support comprising probes attached thereto, means for heating the composition selected in (a) to at least 60 °C during hybridization, and means for cooling the composition selected in (a) during hybridization, wherein each probe comprises a sequence specific for a sequence of a marker for which a forward primer and a reverse primer specific for the locus of said marker were provided in said multiplex real-time PCR in (a),
wherein each probe comprising the same sequence is attached to a specific area of said solid support and each specific area of said solid support is attached to probes comprising the same sequence,
wherein the means for heating the composition selected in (a) comprise a heating device,
wherein the means for cooling the composition selected in (a) comprise a cooling device,
wherein the means for subjecting each first sequence and second sequence which is amplified in the composition selected in (a) to hybridization on a solid support comprise a solid support comprising probes attached thereto, and hybridization reagents, wherein said solid support comprises said pool of probes, wherein each probe of said pool of probes comprises a fluorophore combined with a sequence specific for a sequence of a marker of said human papillomavirus genotype of claim 1, and the probe comprising the fluorophore combined with the sequence specific for said second sequence; and
(c) means for identifying each specific area of said solid support to which a probe is attached and to which a first sequence or second sequence is hybridized, wherein said means comprise an image-capture device;
wherein when the fluorophore of the probe binding to said second sequence is detected and the fluorophore of said pool of probes binding to said first sequence is detected in (a) and at least one specific area of said solid support to which a probe is attached and to which a first sequence is hybridized is identified in (c), said genotype is determined by:
(la) identifying each specific area of said solid support to which a probe is attached and to which a first sequence is hybridized;
(lb) determining which genotype of the human papillomavirus is identified by a marker comprising a sequence specific for a sequence of the probe which is attached to each specific area identified in (la); and
wherein said use comprises subjecting each amplified first sequence and second sequence of the composition selected in (a), to hybridization on a solid support comprising probes attached thereto, wherein during hybridization, the composition selected in (a) is subjected to heating to at least 60 °C and subsequently, to cooling, and wherein, under said hybridization conditions, the amplicons generated in the real-time multiplex PCR hybridize preferentially to the probes in solid phase, which are attached to the solid support, and not to the probes in the liquid phase, which remain from the real-time multiplex PCR.

4. The method according to claim 1, the use of the system according to claim 2, or the use of the kit according to claim 3, wherein said multiplex real-time PCR amplifies:
(i) at least one first sequence of a marker from a genotype of the human papillomavirus which is present in the conserved region of the *HPV L1* or *HPV L2* gene; and
(ii) said second sequence.

5. The method according to claim 1 or 4, the use of the system according to claim 2 or 4, or the use of the kit according to claim 3 or 4, wherein the set of probes defined in (iii) further comprises:
- a probe comprising a fluorophore combined with a sequence specific for a sequence of a marker of the 16 genotype of the human papillomavirus;
- a probe comprising a fluorophore combined with a sequence specific for a sequence of a marker of the 18 genotype of the human papillomavirus.

6. The method according to claim 1 or 4, the use of the system according to any one of claims 2, 4 or 5, or the use of the kit according to any one of claims 3 to 5, wherein the solid support defined in (b) further comprises:
- a probe comprising a sequence specific for a sequence of a marker of the 16 genotype of the human papillomavirus;
- a probe comprising a sequence specific for a sequence of a marker of the 18 genotype of the human papillomavirus.

7. The method, the use of the system, or the use of the kit according to claim 5 or 6, wherein said multiplex real-time PCR amplifies:
(i) at least one first sequence, wherein each first sequence is a sequence of a marker from a human papillomavirus genotype selected from the group consisting of: 16, 18, 31, 33, 35, 39, 45, 51, 52, 56, 58, 59, 66, and 68; and
(ii) said second sequence.

8. The method, the use of the system, or the use of the kit according to claim 5 to 7, wherein the method, the use of the system or the use of the kit is additionally for determining whether said subject is at risk of developing cervical, oral, or anogenital cancer, wherein when any one of the human papillomavirus genotypes selected from the group consisting of: 16, 18, 31, 33, 35, 39, 45, 51, 52, 56, 58, 59, 66, and 68, is determined, said subject is determined to be at risk of developing cervical, oral, or anogenital cancer.

9. The method according to any one of claims 1, or 4 to 8, the use of the system according to any one of claims 2, or 4 to 8, or the use of the kit according to any one of claims 3 to 8, wherein said heating is from 60 to 70°C, or from 60 to 65°C.

10. The method according to any one of claims 1, or 4 to 9, the use of the system according to any one of claims 2, or 4 to 9, or the use of the kit according to any one of claims 3 to 9, wherein said heating is for 1 to 20 minutes, or for 3 to 20 minutes, or for at least 10 minutes.

11. The method according to any one of claims 1, or 4 to 10, the use of the system according to any one of claims 2, or 4 to 10, or the use of the kit according to any one of claims 3 to 10, wherein said cooling consists of cooling to 41 °C.

12. The method according to any one of claims 1, or 4 to 11, the use of the system according to any one of claims 2, or 4 to 11, or the use of the kit according to any one of claims 3 to 11, wherein the forward and/or reverse primer comprises a molecular tag.

13. The method according to any one of claims 1, 4 to 12, the use of the system according to any one of claims 2, 4 to 12, or the use of the kit according to any one of claims 3 to 12, wherein said sample is obtained by means of a sample collection device.

14. The method according to any one of claims 1, 4 to 13, the use of the system according to any one of claims 2, 4 to 13, or the use of the kit according to any one of claims 3 to 13, wherein, prior to hybridization according to (b), the composition selected in (a) is heated to 95 °C for 10 minutes.

15. The use of the system according to any one of claims 2, 4 to 14, or the use of the kit according to any one of claims 3 to 14, wherein the system and/or kit further comprises (aa) means for extracting DNA of human papillomavirus present in a biological sample that has been obtained from a subject.

## Patentansprüche

1. Methode zum Nachweisen und Bestimmen eines humanen Papillomavirus-Genotyps, der mit der Entwicklung von zervikalem, oralem oder anogenitalem Krebs bei einem Patienten verbunden ist, wobei der humane Papillomavirus-Genotyp aus der Gruppe ausgewählt ist, bestehend aus: 31, 33, 35, 39, 45, 51, 52, 56, 58, 59, 66 und 68, das Verfahren umfassend:
(a) Unterziehen mindestens einer in DNA enthaltenen Sequenz, die in einer von dem Patienten erhaltenen biologischen Probe vorhanden ist, einer Multiplex-Echtzeit-PCR, wobei die DNA aus der biologischen Probe extrahiert wird, bevor sie einer Multiplex-Echtzeit-PCR unterzogen wird, die amplifiziert:
(i) mindestens eine erste Sequenz eines Markers eines Genotyps des humanen Papillomavirus, wobei die Amplifikation der ersten Sequenz unter Verwendung eines Vorwärtsprimers und eines Rückwärtsprimers, die für die Stelle des Markers spezifisch sind, durchgeführt wird; und
(ii) eine zweite Sequenz eines Markers eines menschlichen Housekeeping-Gens, wobei die Amplifikation der zweiten Sequenz unter Verwendung eines Vorwärtsprimers und eines Rückwärtsprimers, der für die Stelle des Markers spezifisch ist, durchgeführt wird,
in einer Zusammensetzung, umfassend:
(iii) einen Satz von Sonden, der einen Pool von Sonden umfasst, wobei jede Sonde des Pools von Sonden ein Fluorophor umfasst, das mit einer Sequenz kombiniert ist, die für eine Sequenz eines Markers eines humanen Papillomavirus-Genotyps spezifisch ist, der aus der Gruppe ausgewählt ist, bestehend aus: 31, 33, 35, 39, 45, 51, 52, 56, 58, 59, 66 und 68, wobei jeder humane Papillomavirus-Genotyp einen Marker aufweist, der eine Sequenz umfasst, die für mindestens eine Sequenz einer Sonde des Sondenpools spezifisch ist; und
(iv) eine Sonde, die ein Fluorophor in Kombination mit einer für die zweite Sequenz spezifischen Sequenz umfasst,
wobei jede Sonde, die an die Sequenz eines dafür spezifischen Markers bindet, detektiert wird, wobei, wenn:
- der Fluorophor der Sonde, der an die zweite Sequenz bindet, nicht nachgewiesen wird und der Fluorophor des Sondenpools, der an die erste Sequenz bindet, nicht nachgewiesen wird, Schritt (a) für den Patienten wiederholt wird;
- der an die zweite Sequenz bindende Fluorophor der Sonde nachgewiesen wird und der an die erste Sequenz bindende Fluorophor des Pools von Sonden nachgewiesen wird, ein humaner Papillomavirus-Genotyp, der mit der Entwicklung von zervikalem, oralem oder anogenitalem Krebs verbunden ist, nachgewiesen wird und die Zusammensetzung ausgewählt wird, die die amplifizierte erste Sequenz und die zweite Sequenz umfasst;
(b) Unterziehen jeder amplifizierten ersten Sequenz und zweiten Sequenz der in Schritt (a) ausgewählten Zusammensetzung einer Hybridisierung auf einem festen Träger, der daran gebundene Sonden umfasst, wobei während der Hybridisierung die in Schritt (a) ausgewählte Zusammensetzung einer Erwärmung auf mindestens 60 °C und anschließend einer allmählichen Abkühlung unterzogen wird, wobei unter den Hybridisierungsbedingungen die im vorherigen Schritt (a) erzeugten Amplikons vorzugsweise an die Sonden in fester Phase hybridisieren, die an den festen Träger gebunden sind, und nicht an die Sonden in flüssiger Phase, die aus der Echtzeit-Multiplex-PCR von Schritt (a) verbleiben, wobei jede Sonde eine Sequenz umfasst, die für eine Sequenz eines Markers spezifisch ist, für den in der Multiplex-Echtzeit-PCR in Schritt (a) ein Vorwärtsprimer und ein Rückwärtsprimer bereitgestellt wurden, die für den Ort des Markers spezifisch sind,
wobei jede Sonde, die die gleiche Sequenz umfasst, an einen spezifischen Bereich des festen Trägers gebunden ist und jeder spezifische Bereich des festen Trägers an Sonden gebunden ist, die die gleiche Sequenz umfassen; und
(c) Identifizieren jedes spezifischen Bereiches des festen Trägers, an dem eine Sonde angebracht ist und an den eine erste Sequenz oder zweite Sequenz hybridisiert ist;
wobei, wenn das Fluorophor der Sonde, die an die zweite Sequenz bindet, nachgewiesen wird und das Fluorophor des Pools von Sonden, die an die erste Sequenz binden, in Schritt (a) nachgewiesen wird und mindestens ein spezifischer Bereich des festen Trägers, an den eine Sonde gebunden ist und an den eine erste Sequenz hybridisiert ist, in Schritt (c) identifiziert wird, der Genotyp bestimmt wird durch:
(la) Identifizieren jedes spezifischen Bereiches des festen Trägers, an dem eine Sonde angebracht ist und an den eine erste Sequenz hybridisiert ist;
(Ib) Bestimmen, welcher Genotyp des humanen Papillomavirus durch einen Marker identifiziert wird, der eine für eine Sequenz der Sonde spezifische Sequenz umfasst, die an jeden in Schritt (la) identifizierten spezifischen Bereich gebunden ist.

2. Verwendung eines Systems zum Nachweisen und Bestimmen eines humanen Papillomavirus-Genotyps, der mit der Entwicklung von zervikalem, oralem oder anogenitalem Krebs bei einem Patienten verbunden ist, wobei der humane Papillomavirus-Genotyp aus der Gruppe ausgewählt ist, bestehend aus: 31, 33, 35, 39, 45, 51, 52, 56, 58, 59, 66 und 68, das System umfassend:
a) Mittel zum Unterziehen mindestens einer in DNA enthaltenen Sequenz, die in einer von dem Patienten erhaltenen biologischen Probe vorhanden ist, einer Multiplex-Echtzeit-PCR, die mindestens eine erste Sequenz eines Markers eines Genotyps des humanen Papillomavirus nach Anspruch 1 und die zweite Sequenz eines Markers eines humanen Housekeeping-Gens nach Anspruch 1 amplifiziert,
in einer Zusammensetzung, die den Pool von Sonden nach Anspruch 1 umfasst und wobei die Sonde einen Fluorophor umfasst, der mit einer für die zweite Sequenz nach Anspruch 1 spezifischen Sequenz kombiniert ist,
wobei jede Sonde, die an die Sequenz eines dafür spezifischen Markers bindet, detektiert wird, wobei, wenn
- der an die zweite Sequenz bindende Fluorophor der Sonde nachgewiesen wird und der an die erste Sequenz bindende Fluorophor des Pools von Sonden nachgewiesen wird, ein mit der Entwicklung von zervikalem, oralem oder anogenitalem Krebs assoziierter humaner Papillomavirus-Genotyp nachgewiesen wird und die Zusammensetzung aus der amplifizierten ersten Sequenz und der zweiten Sequenz ausgewählt wird, und
wobei die Mittel zum Unterziehen mindestens einer Sequenz dem Multiplexen in Echtzeit PCR Folgendes umfassen: ein Multiplex-Echtzeit-PCR-Instrument; den Vorwärtsprimer und den Rückwärtsprimer, die für die Stelle der mindestens einen ersten Sequenz spezifisch sind; den Vorwärtsprimer und den Rückwärtsprimer, die für die Stelle der für die zweite Sequenz spezifischen Sequenz spezifisch sind; den Pool von Sonden, wobei jede Sonde des Pools von Sonden einen Fluorophor umfasst, der mit einer Sequenz kombiniert ist, die für eine Sequenz eines Markers eines humanen Papillomavirus-Genotyps nach Anspruch 1 spezifisch ist; und die Sonde den Fluorophor umfasst, der mit der für die zweite Sequenz spezifischen Sequenz kombiniert ist; und Multiplex-Echtzeit-PCR-Reagenzien;
(b) Mittel zum Unterziehen jeder amplifizierten ersten Sequenz und zweiten Sequenz der unter (a) ausgewählten Zusammensetzung einer Hybridisierung auf einem festen Träger, der daran angebrachte Sonden umfasst, Mittel zum Erwärmen der unter (a) ausgewählten Zusammensetzung während der Hybridisierung auf mindestens 60 °C und Mittel zum Abkühlen der unter (a) ausgewählten Zusammensetzung während der Hybridisierung, wobei jede Sonde eine Sequenz umfasst, die für eine Sequenz eines Markers spezifisch ist, für die in der Multiplex-Echtzeit-PCR in (a) ein Vorwärtsprimer und ein Rückwärtsprimer bereitgestellt wurden, die für den Ort des Markers spezifisch sind,
wobei jede Sonde, die die gleiche Sequenz umfasst, an einem spezifischen Bereich des festen Trägers befestigt ist und jeder bestimmte Bereich des festen Trägers an Sonden befestigt ist, die die gleiche Sequenz umfassen,
wobei die Mittel zum Erwärmen der in (a) ausgewählten Zusammensetzung eine Heizvorrichtung umfassen,
wobei die Mittel zum Abkühlen der in (a) ausgewählten Zusammensetzung eine Kühlvorrichtung umfassen, wobei die Mittel zum Unterziehen jeder ersten Sequenz und zweiten Sequenz, die in der in (a) ausgewählten Zusammensetzung amplifiziert sind, einer Hybridisierung auf einem festen Träger einen festen Träger umfassen, der daran gebundene Sonden und Hybridisierungsreagenzien umfasst, wobei der feste Träger den Pool von Sonden umfasst, wobei jede Sonde des Pools von Sonden einen Fluorophor umfasst, der mit einer Sequenz kombiniert ist, die für eine Sequenz eines Markers des humanen Papillomavirus-Genotyps nach Anspruch 1 spezifisch ist; und die Sonde den Fluorophor umfasst, der mit der für die zweite Sequenz spezifischen Sequenz kombiniert ist; und
(c) Mittel zum Identifizieren jedes spezifischen Bereiches des festen Trägers, an dem eine Sonde angebracht ist und an den eine erste Sequenz oder zweite Sequenz hybridisiert ist, wobei die Mittel eine Bilderfassungsvorrichtung umfassen;
wobei, wenn der Fluorophor der Sonde, der an die zweite Sequenz bindet, nachgewiesen wird und der Fluorophor des Pools von Sonden, der an die erste Sequenz bindet, in (a) nachgewiesen wird und mindestens ein spezifischer Bereich des festen Trägers, an den eine Sonde gebunden ist und an den eine erste Sequenz hybridisiert ist, in (c) identifiziert wird, der Genotyp bestimmt wird durch:
(la) Identifizieren jedes spezifischen Bereiches des festen Trägers, an dem eine Sonde angebracht ist und an den eine erste Sequenz hybridisiert ist;
(Ib) Bestimmen, welcher Genotyp des humanen Papillomavirus durch einen Marker identifiziert wird, der eine für eine Sequenz der Sonde spezifische Sequenz umfasst, die an jeden in (la) identifizierten spezifischen Bereich gebunden ist; und
wobei die Verwendung umfasst, dass jede amplifizierte erste Sequenz und zweite Sequenz der in (a) ausgewählten Zusammensetzung einer Hybridisierung auf einem festen Träger unterzogen wird, der daran gebundene Sonden umfasst, wobei während der Hybridisierung die in (a) ausgewählte Zusammensetzung einer Erwärmung auf mindestens 60 °C und anschließend einer Abkühlung unterzogen wird, und wobei unter den Hybridisierungsbedingungen die in der Echtzeit-Multiplex-PCR erzeugten Amplikons vorzugsweise an die Sonden in fester Phase hybridisieren, die an den festen Träger gebunden sind, und nicht an die Sonden in der flüssigen Phase, die aus der Echtzeit-Multiplex-PCR verbleiben.

3. Verwendung eines Kits zum Nachweisen und Bestimmen eines humanen Papillomavirus-Genotyps, der mit der Entwicklung von zervikalem, oralem oder anogenitalem Krebs bei einem Patienten verbunden ist, wobei der humane Papillomavirus-Genotyp aus der Gruppe ausgewählt ist, bestehend aus: 31, 33, 35, 39, 45, 51,52, 56, 58, 59, 66 und 68, wobei das Kit umfasst:
a) Mittel zum Unterziehen mindestens einer in DNA enthaltenen Sequenz, die in einer von dem Patienten erhaltenen biologischen Probe vorhanden ist, einer Multiplex-Echtzeit-PCR, die mindestens eine erste Sequenz eines Markers eines Genotyps des humanen Papillomavirus nach Anspruch 1 und die zweite Sequenz eines Markers eines humanen Housekeeping-Gens nach Anspruch 1 amplifiziert,
in einer Zusammensetzung, die den Pool von Sonden nach Anspruch 1 umfasst und wobei die Sonde einen Fluorophor umfasst, der mit einer für die zweite Sequenz nach Anspruch 1 spezifischen Sequenz kombiniert ist,
wobei jede Sonde, die an die Sequenz eines dafür spezifischen Markers bindet, detektiert wird, wobei, wenn
- der an die zweite Sequenz bindende Fluorophor der Sonde nachgewiesen wird und der an die erste Sequenz bindende Fluorophor des Pools von Sonden nachgewiesen wird, ein mit der Entwicklung von zervikalem, oralem oder anogenitalem Krebs assoziierter humaner Papillomavirus-Genotyp nachgewiesen wird und die Zusammensetzung aus der amplifizierten ersten Sequenz und der zweiten Sequenz ausgewählt wird, und
wobei die Mittel zum Unterziehen mindestens einer Sequenz einer Multiplex-Echtzeit-PCR umfassen; ein Multiplex-Echtzeit-PCR-Instrument; den Vorwärtsprimer und den Rückwärtsprimer, die für den Ort der mindestens einen ersten Sequenz spezifisch sind; den Vorwärtsprimer und den Rückwärtsprimer, die für den Ort der für die zweite Sequenz spezifischen Sequenz spezifisch sind; den Pool von Sonden, wobei jede Sonde des Pools von Sonden einen Fluorophor umfasst, der mit einer Sequenz kombiniert ist, die für eine Sequenz eines Markers eines humanen Papillomavirus-Genotyps nach Anspruch 1 spezifisch ist, und die Sonde den Fluorophor umfasst, der mit der für die zweite Sequenz spezifischen Sequenz kombiniert ist; und Multiplex-Echtzeit-PCR-Reagenzien;
(b) Mittel zum Unterziehen jeder amplifizierten ersten Sequenz und zweiten Sequenz der unter (a) ausgewählten Zusammensetzung einer Hybridisierung auf einem festen Träger, der daran angebrachte Sonden umfasst, Mittel zum Erwärmen der unter (a) ausgewählten Zusammensetzung während der Hybridisierung auf mindestens 60 °C und Mittel zum Abkühlen der unter (a) ausgewählten Zusammensetzung während der Hybridisierung, wobei jede Sonde eine Sequenz umfasst, die für eine Sequenz eines Markers spezifisch ist, für die in der Multiplex-Echtzeit-PCR in (a) ein Vorwärtsprimer und ein Rückwärtsprimer bereitgestellt wurden, die für den Ort des Markers spezifisch sind,
wobei jede Sonde, die die gleiche Sequenz umfasst, an einem spezifischen Bereich des festen Trägers befestigt ist und jeder bestimmte Bereich des festen Trägers an Sonden befestigt ist, die die gleiche Sequenz umfassen,
wobei die Mittel zum Erwärmen der in (a) ausgewählten Zusammensetzung eine Heizvorrichtung umfassen,
wobei die Mittel zum Kühlen der in (a) ausgewählten Zusammensetzung eine Kühlvorrichtung umfassen, wobei die Mittel zum Unterziehen jeder ersten Sequenz und zweiten Sequenz, die in der in (a) ausgewählten Zusammensetzung amplifiziert sind, einer Hybridisierung auf einem festen Träger einen festen Träger umfassen, der daran gebundene Sonden und Hybridisierungsreagenzien umfasst, wobei der feste Träger den Pool von Sonden umfasst, wobei jede Sonde des Pools von Sonden einen Fluorophor umfasst, der mit einer Sequenz kombiniert ist, die für eine Sequenz eines Markers des humanen Papillomavirus-Genotyps nach Anspruch 1 spezifisch ist, und die Sonde den Fluorophor umfasst, der mit der für die zweite Sequenz spezifischen Sequenz kombiniert ist; und
(c) Mittel zum Identifizieren jedes spezifischen Bereiches des festen Trägers, an dem eine Sonde angebracht ist und an den eine erste Sequenz oder zweite Sequenz hybridisiert ist, wobei die Mittel eine Bilderfassungsvorrichtung umfassen;
wobei, wenn der Fluorophor der Sonde, der an die zweite Sequenz bindet, nachgewiesen wird und der Fluorophor des Pools von Sonden, der an die erste Sequenz bindet, in (a) nachgewiesen wird und mindestens ein spezifischer Bereich des festen Trägers, an den eine Sonde gebunden ist und an den eine erste Sequenz hybridisiert ist, in (c) identifiziert wird, der Genotyp bestimmt wird durch:
(la) Identifizieren jedes spezifischen Bereiches des festen Trägers, an dem eine Sonde angebracht ist und an den eine erste Sequenz hybridisiert ist;
(Ib) Bestimmen, welcher Genotyp des humanen Papillomavirus durch einen Marker identifiziert wird, der eine für eine Sequenz der Sonde spezifische Sequenz umfasst, die an jeden in (la) identifizierten spezifischen Bereich gebunden ist; und
wobei die Verwendung umfasst, dass jede amplifizierte erste Sequenz und zweite Sequenz der in (a) ausgewählten Zusammensetzung einer Hybridisierung auf einem festen Träger unterzogen wird, der daran gebundene Sonden umfasst, wobei während der Hybridisierung die in (a) ausgewählte Zusammensetzung einer Erwärmung auf mindestens 60 °C und anschließend einer Abkühlung unterzogen wird, und wobei unter den Hybridisierungsbedingungen die in der Echtzeit-Multiplex-PCR erzeugten Amplikons vorzugsweise an die Sonden in fester Phase hybridisieren, die an den festen Träger gebunden sind, und nicht an die Sonden in der flüssigen Phase, die aus der Echtzeit-Multiplex-PCR verbleiben.

4. Methode nach Anspruch 1, die Verwendung des Systems nach Anspruch 2 oder die Verwendung des Kits nach Anspruch 3, wobei die Multiplex-Echtzeit-PCR amplifiziert:
(i) mindestens eine erste Sequenz eines Markers aus einem Genotyp des humanen Papillomavirus, der in der konservierten Region des *HPV-L1-* oder *HPV-L2-Gens* vorhanden ist; und
(ii) die zweite Sequenz.

5. Methode nach Anspruch 1 oder 4, die Verwendung des Systems nach Anspruch 2 oder 4 oder die Verwendung des Kits nach Anspruch 3 oder 4, wobei der Satz von Sonden, der in (iii) definiert ist, ferner umfasst:
- eine Sonde, die ein Fluorophor in Kombination mit einer Sequenz umfasst, die für eine Sequenz eines Markers des Genotyps 16 des humanen Papillomavirus spezifisch ist;
- eine Sonde, die ein Fluorophor kombiniert mit einer Sequenz umfasst, die für eine Sequenz eines Markers des Genotyps 18 des humanen Papillomavirus spezifisch ist.

6. Methode nach Anspruch 1 oder 4, die Verwendung des Systems nach einem der Ansprüche 2, 4 oder 5 oder die Verwendung des Kits nach einem der Ansprüche 3 bis 5, wobei der in (b) definierte feste Träger ferner umfasst:
- eine Sonde, die eine Sequenz umfasst, die für eine Sequenz eines Markers des Genotyps 16 des humanen Papillomavirus spezifisch ist;
- eine Sonde, die eine Sequenz umfasst, die für eine Sequenz eines Markers des Genotyps 18 des humanen Papillomavirus spezifisch ist.

7. Methode, die Verwendung des Systems oder die Verwendung des Kits nach Anspruch 5 oder 6, wobei die Multiplex-Echtzeit-PCR amplifiziert:
(i) mindestens eine erste Sequenz, wobei jede erste Sequenz eine Sequenz eines Markers aus einem humanen Papillomavirus-Genotyp ist, der aus der Gruppe ausgewählt ist, bestehend aus: 16, 18, 31, 33, 35, 39, 45, 51, 52, 56, 58, 59, 66 und 68; und
(ii) die zweite Sequenz.

8. Verfahren, die Verwendung des Systems oder die Verwendung des Kits nach Anspruch 5 bis 7, wobei das Verfahren, die Verwendung des Systems oder die Verwendung des Kits zusätzlich dem Bestimmen dient, ob der Patient gefährdet ist, zervikalen, oralen oder anogenitalen Krebs zu entwickeln, wobei, wenn einer der humanen Papillomavirus-Genotypen ausgewählt aus der Gruppe bestehend aus: 16, 18, 31, 33, 35, 39, 45, 51, 52, 56, 58, 59, 66 und 68 bestimmt wird, festgestellt wird, dass der Patient einem Risiko für die Entwicklung von zervikalem, oralem oder anogenitalem Krebs ausgesetzt ist.

9. Methode nach einem der Ansprüche 1 oder 4 bis 8, die Verwendung des Systems nach einem der Ansprüche 2 oder 4 bis 8 oder die Verwendung des Kits nach einem der Ansprüche 3 bis 8, wobei das Erhitzen 60 bis 70 °C oder 60 bis 65 °C beträgt.

10. Methode nach einem der Ansprüche 1 oder 4 bis 9, die Verwendung des Systems nach einem der Ansprüche 2 oder 4 bis 9 oder die Verwendung des Kits nach einem der Ansprüche 3 bis 9, wobei das Erhitzen 1 bis 20 Minuten oder 3 bis 20 Minuten oder mindestens 10 Minuten dauert.

11. Methode nach einem der Ansprüche 1 oder 4 bis 10, die Verwendung des Systems nach einem der Ansprüche 2 oder 4 bis 10 oder die Verwendung des Kits nach einem der Ansprüche 3 bis 10, wobei das Abkühlen aus einem Abkühlen auf 41 °C besteht.

12. Methode nach einem der Ansprüche 1 oder 4 bis 11, die Verwendung des Systems nach einem der Ansprüche 2 oder 4 bis 11 oder die Verwendung des Kits nach einem der Ansprüche 3 bis 11, wobei der Vorwärts- und/oder Rückwärtsprimer einen molekularen Tag umfassen.

13. Methode nach einem der Ansprüche 1, 4 bis 12, die Verwendung des Systems nach einem der Ansprüche 2, 4 bis 12 oder die Verwendung des Kits nach einem der Ansprüche 3 bis 12, wobei die Probe mittels einer Probenentnahmevorrichtung erhalten wird.

14. Methode nach einem der Ansprüche 1, 4 bis 13, Verwendung des Systems nach einem der Ansprüche 2, 4 bis 13 oder Verwendung des Kits nach einem der Ansprüche 3 bis 13, wobei vor der Hybridisierung nach (b) die in (a) ausgewählte Zusammensetzung 10 Minuten lang auf 95 °C erhitzt wird.

15. Verwendung des Systems nach einem der Ansprüche 2, 4 bis 14 oder die Verwendung des Kits nach einem der Ansprüche 3 bis 14, wobei das System und/oder das Kit ferner (aa) Mittel zum Extrahieren von DNA von humanem Papillomavirus, das in einer biologischen Probe vorhanden ist, die von einem Patienten erhalten wurde, umfasst.

## Revendications

1. Une méthode pour détecter et déterminer un génotype du papillomavirus humain associé au développement d'un cancer du col de l'utérus, de la bouche ou anogénital chez un sujet, dans laquelle ledit génotype du papillomavirus humain est choisi dans le groupe constitué de : 31, 33, 35, 39, 45, 51, 52, 56, 58, 59, 66 et 68, la méthode consistant à :
(a) soumettre au moins une séquence comprise dans l'ADN présent dans un échantillon biologique qui a été obtenu auprès dudit sujet à une PCR multiplex en temps réel, ledit ADN étant extrait dudit échantillon biologique avant d'être soumis à une PCR multiplex en temps réel qui amplifie :
(i) au moins une première séquence d'un marqueur d'un génotype du papillomavirus humain, l'amplification de ladite première séquence étant effectuée à l'aide d'une amorce directe et d'une amorce inverse, spécifiques du locus dudit marqueur ; et
(ii) une deuxième séquence d'un marqueur d'un gène domestique humain, l'amplification de ladite deuxième séquence étant effectuée à l'aide d'une amorce directe et d'une amorce inverse, spécifiques du locus dudit marqueur,
dans une composition comprenant :
(iii) un ensemble de sondes comprenant un groupe de sondes, chaque sonde dudit groupe de sondes comprenant un fluorophore combiné à une séquence spécifique d'une séquence d'un marqueur d'un génotype du papillomavirus humain choisi dans le groupe constitué de : 31, 33, 35, 39, 45, 51, 52, 56, 58, 59, 66 et 68, chaque génotype du papillomavirus humain ayant un marqueur comprenant une séquence qui est spécifique d'au moins une séquence d'une sonde dudit groupe de sondes ; et
(iv) une sonde comprenant un fluorophore combiné à une séquence spécifique de ladite deuxième séquence,
dans laquelle chaque sonde qui se lie à la séquence d'un marqueur qui lui est spécifique est détectée, dans laquelle :
- lorsque le fluorophore de la sonde se liant à ladite deuxième séquence n'est pas détecté et que le fluorophore dudit groupe de sondes se liant à ladite première séquence n'est pas détecté, l'étape (a) est répétée pour ledit sujet ;
- lorsque le fluorophore de la sonde se liant à ladite deuxième séquence est détecté et que le fluorophore dudit groupe de sondes se liant à ladite première séquence est détecté, un génotype du papillomavirus humain associé au développement d'un cancer du col de l'utérus, de la bouche ou anogénital est détecté et la composition comprenant la première séquence et la deuxième séquence amplifiées est sélectionnée ;
(b) soumettre chaque première séquence et deuxième séquence amplifiées de la composition sélectionnée à l'étape (a) à une hybridation sur un support solide comprenant des sondes qui y sont fixées, dans laquelle lors de l'hybridation, la composition sélectionnée à l'étape (a) est soumise à un chauffage à au moins 60 °C et ensuite à un refroidissement progressif, dans laquelle, dans lesdites conditions d'hybridation, les amplicons générés à l'étape précédente (a) s'hybrident préférentiellement aux sondes en phase solide, qui sont fixées au support solide, et non aux sondes en phase liquide, qui restent suite à la PCR multiplex en temps réel de l'étape (a), dans laquelle chaque sonde comprend une séquence spécifique d'une séquence d'un marqueur pour lequel une amorce directe et une amorce inverse spécifiques du locus dudit marqueur ont été fournies dans ladite PCR multiplex en temps réel à l'étape (a),
dans laquelle chaque sonde comprenant la même séquence est fixée à une zone spécifique dudit support solide et chaque zone spécifique dudit support solide est fixée à des sondes comprenant la même séquence ; et
(c) identifier chaque zone spécifique dudit support solide à laquelle une sonde est fixée et à laquelle une première séquence ou une deuxième séquence est hybridée ;
dans laquelle lorsque le fluorophore de la sonde se liant à ladite deuxième séquence est détecté et que le fluorophore dudit groupe de sondes se liant à ladite première séquence est détecté à l'étape (a) et qu'au moins une zone spécifique dudit support solide à laquelle une sonde est fixée et à laquelle une première séquence est hybridée est identifiée à l'étape (c), ledit génotype est déterminé par :
(la) identifier chaque zone spécifique dudit support solide à laquelle une sonde est fixée et à laquelle une première séquence est hybridée ;
(Ib) déterminer le génotype du papillomavirus humain qui est identifié par un marqueur comprenant une séquence spécifique d'une séquence de la sonde qui est fixée à chaque zone spécifique identifiée à l'étape (la).

2. Utilisation d'une méthode pour détecter et déterminer un génotype du papillomavirus humain associé au développement d'un cancer du col de l'utérus, de la bouche ou anogénital chez un sujet, dans laquelle ledit génotype du papillomavirus humain est choisi dans le groupe constitué de : 31, 33, 35, 39, 45, 51, 52, 56, 58, 59, 66 et 68, le système comprenant :
(a) des moyens pour soumettre au moins une séquence comprise dans l'ADN présent dans un échantillon biologique obtenu auprès dudit sujet à une PCR multiplex en temps réel qui amplifie au moins une première séquence d'un marqueur d'un génotype du papillomavirus humain de la revendication 1 et la deuxième séquence d'un marqueur d'un gène domestique humain de la revendication 1,
dans une composition comprenant le groupe de sondes de la revendication 1 et la sonde comprenant un fluorophore combiné à une séquence spécifique de ladite deuxième séquence de la revendication 1,
dans laquelle chaque sonde qui se lie à la séquence d'un marqueur qui lui est spécifique est détectée, dans laquelle :
- lorsque le fluorophore de la sonde se liant à ladite deuxième séquence est détecté et que le fluorophore dudit groupe de sondes se liant à ladite première séquence est détecté, un génotype du papillomavirus humain associé au développement d'un cancer du col de l'utérus, de la bouche ou anogénital est détecté et la composition comprenant la première séquence amplifiée et la deuxième séquence est sélectionnée, et
dans laquelle les moyens pour soumettre au moins une séquence à une PCR multiplex en temps réel comprennent : un instrument de PCR multiplex en temps réel ; l'amorce directe et l'amorce inverse, spécifiques du locus de ladite au moins une première séquence ; l'amorce directe et l'amorce inverse, spécifiques du locus de la séquence spécifique de ladite deuxième séquence ; le groupe de sondes, chaque sonde dudit groupe de sondes comprenant un fluorophore combiné à une séquence spécifique d'une séquence d'un marqueur d'un génotype du papillomavirus humain de la revendication 1 ; et la sonde comprenant le fluorophore combiné à la séquence spécifique de ladite deuxième séquence ; et des réactifs de PCR multiplex en temps réel ;
(b) des moyens pour soumettre chaque première séquence et deuxième séquence amplifiées de la composition sélectionnée en (a) à une hybridation sur un support solide comprenant des sondes qui y sont fixées, des moyens pour chauffer la composition sélectionnée en (a) à au moins 60 °C pendant l'hybridation, et des moyens pour refroidir la composition sélectionnée en (a) pendant l'hybridation, dans laquelle chaque sonde comprend une séquence spécifique d'une séquence d'un marqueur pour lequel une amorce directe et une amorce inverse spécifiques du locus dudit marqueur ont été fournies dans ladite PCR multiplex en temps réel en (a),
dans laquelle chaque sonde comprenant la même séquence est fixée à une zone spécifique dudit support solide et chaque zone spécifique dudit support solide est fixée à des sondes comprenant la même séquence,
dans laquelle les moyens pour chauffer la composition sélectionnée en (a) comprennent un dispositif de chauffage,
dans laquelle les moyens pour refroidir la composition sélectionnée en (a) comprennent un dispositif de refroidissement, dans laquelle les moyens pour soumettre chaque première séquence et deuxième séquence qui sont amplifiées dans la composition sélectionnée en (a) à une hybridation sur un support solide comprennent un support solide comprenant des sondes qui y sont fixées, et des réactifs d'hybridation, dans laquelle ledit support solide comprend ledit groupe de sondes, dans laquelle chaque sonde dudit groupe de sondes comprend un fluorophore combiné à une séquence spécifique d'une séquence d'un marqueur dudit génotype du papillomavirus humain de la revendication 1 ; et la sonde comprenant le fluorophore combiné à la séquence spécifique de ladite deuxième séquence ; et
(c) des moyens pour identifier chaque zone spécifique dudit support solide à laquelle une sonde est fixée et à laquelle une première séquence ou une deuxième séquence est hybridée, dans laquelle lesdits moyens comprennent un dispositif de capture d'image ;
dans laquelle lorsque le fluorophore de la sonde se liant à ladite deuxième séquence est détecté et que le fluorophore dudit groupe de sondes se liant à ladite première séquence est détecté en (a) et qu'au moins une zone spécifique dudit support solide à laquelle une sonde est fixée et à laquelle une première séquence est hybridée est identifiée en (c), ledit génotype est déterminé par :
(la) identifier chaque zone spécifique dudit support solide à laquelle une sonde est fixée et à laquelle une première séquence est hybridée ;
(Ib) déterminer le génotype du papillomavirus humain qui est identifié par un marqueur comprenant une séquence spécifique d'une séquence de la sonde qui est fixée à chaque zone spécifique identifiée en (la) ; et
dans laquelle ladite utilisation consiste à soumettre chaque première séquence et deuxième séquence amplifiées de la composition sélectionnée en (a) à une hybridation sur un support solide comprenant des sondes qui y sont fixées, dans laquelle pendant l'hybridation, la composition sélectionnée en (a) est soumise à un chauffage à au moins 60 °C et ensuite à un refroidissement, et dans laquelle, dans lesdites conditions d'hybridation, les amplicons générés dans la PCR multiplex en temps réel s'hybrident préférentiellement aux sondes en phase solide, qui sont fixées au support solide, et non aux sondes en phase liquide, qui restent suite à la PCR multiplex en temps réel.

3. Utilisation d'un kit pour détecter et déterminer un génotype du papillomavirus humain associé au développement d'un cancer du col de l'utérus, de la bouche ou anogénital chez un sujet, dans laquelle ledit génotype du papillomavirus humain est choisi dans le groupe constitué de : 31, 33, 35, 39, 45, 51, 52, 56, 58, 59, 66 et 68, le kit comprenant :
(a) des moyens pour soumettre au moins une séquence comprise dans l'ADN présent dans un échantillon biologique obtenu auprès dudit sujet à une PCR multiplex en temps réel qui amplifie au moins une première séquence d'un marqueur d'un génotype du papillomavirus humain de la revendication 1 et la deuxième séquence d'un marqueur d'un gène domestique humain de la revendication 1,
dans une composition comprenant le groupe de sondes de la revendication 1 et la sonde comprenant un fluorophore combiné à une séquence spécifique de ladite deuxième séquence de la revendication 1,
dans laquelle chaque sonde qui se lie à la séquence d'un marqueur qui lui est spécifique est détectée, dans laquelle :
- lorsque le fluorophore de la sonde se liant à ladite deuxième séquence est détecté et que le fluorophore dudit groupe de sondes se liant à ladite première séquence est détecté, un génotype du papillomavirus humain associé au développement d'un cancer du col de l'utérus, de la bouche ou anogénital est détecté et la composition comprenant la première séquence amplifiée et la deuxième séquence est sélectionnée, et
dans laquelle les moyens pour soumettre au moins une séquence à une PCR multiplex en temps réel comprennent : un instrument de PCR multiplex en temps réel ; l'amorce directe et l'amorce inverse, spécifiques du locus de ladite au moins une première séquence ; l'amorce directe et l'amorce inverse, spécifiques du locus de la séquence spécifique de ladite deuxième séquence ; le groupe de sondes, chaque sonde dudit groupe de sondes comprenant un fluorophore combiné à une séquence spécifique d'une séquence d'un marqueur d'un génotype du papillomavirus humain de la revendication 1, et la sonde comprenant le fluorophore combiné à la séquence spécifique de ladite deuxième séquence ; et des réactifs de PCR multiplex en temps réel ;
(b) des moyens pour soumettre chaque première séquence et deuxième séquence amplifiées de la composition sélectionnée en (a) à une hybridation sur un support solide comprenant des sondes qui y sont fixées, des moyens pour chauffer la composition sélectionnée en (a) à au moins 60 °C pendant l'hybridation, et des moyens pour refroidir la composition sélectionnée en (a) pendant l'hybridation, dans laquelle chaque sonde comprend une séquence spécifique d'une séquence d'un marqueur pour lequel une amorce directe et une amorce inverse spécifiques du locus dudit marqueur ont été fournies dans ladite PCR multiplex en temps réel en (a),
dans laquelle chaque sonde comprenant la même séquence est fixée à une zone spécifique dudit support solide et chaque zone spécifique dudit support solide est fixée à des sondes comprenant la même séquence,
dans laquelle les moyens pour chauffer la composition sélectionnée en (a) comprennent un dispositif de chauffage,
dans laquelle les moyens pour refroidir la composition sélectionnée en (a) comprennent un dispositif de refroidissement, dans laquelle les moyens pour soumettre chaque première séquence et deuxième séquence qui sont amplifiées dans la composition sélectionnée en (a) à une hybridation sur un support solide comprennent un support solide comprenant des sondes qui y sont fixées, et des réactifs d'hybridation, dans laquelle ledit support solide comprend ledit groupe de sondes, dans laquelle chaque sonde dudit groupe de sondes comprend un fluorophore combiné à une séquence spécifique d'une séquence d'un marqueur dudit génotype du papillomavirus humain de la revendication 1, et la sonde comprenant le fluorophore combiné à la séquence spécifique de ladite deuxième séquence ; et
(c) des moyens pour identifier chaque zone spécifique dudit support solide à laquelle une sonde est fixée et à laquelle une première séquence ou une deuxième séquence est hybridée, dans laquelle lesdits moyens comprennent un dispositif de capture d'image ;
dans laquelle lorsque le fluorophore de la sonde se liant à ladite deuxième séquence est détecté et que le fluorophore dudit groupe de sondes se liant à ladite première séquence est détecté en (a) et qu'au moins une zone spécifique dudit support solide à laquelle une sonde est fixée et à laquelle une première séquence est hybridée est identifiée en (c), ledit génotype est déterminé par :
(la) identifier chaque zone spécifique dudit support solide à laquelle une sonde est fixée et à laquelle une première séquence est hybridée ;
(Ib) déterminer le génotype du papillomavirus humain qui est identifié par un marqueur comprenant une séquence spécifique d'une séquence de la sonde qui est fixée à chaque zone spécifique identifiée en (la) ;
dans laquelle ladite utilisation consiste à soumettre chaque première séquence et deuxième séquence amplifiées de la composition sélectionnée en (a) à une hybridation sur un support solide comprenant des sondes qui y sont fixées, dans laquelle pendant l'hybridation, la composition sélectionnée en (a) est soumise à un chauffage à au moins 60 °C et ensuite à un refroidissement, et dans laquelle, dans lesdites conditions d'hybridation, les amplicons générés dans la PCR multiplex en temps réel s'hybrident préférentiellement aux sondes en phase solide, qui sont fixées au support solide, et non aux sondes en phase liquide, qui restent suite à la PCR multiplex en temps réel.

4. La méthode selon la revendication 1, l'utilisation du système selon la revendication 2, ou l'utilisation du kit selon la revendication 3, dans laquelle ladite PCR multiplexe en temps réel amplifie :
(i) au moins une première séquence d'un marqueur d'un génotype du papillomavirus humain qui est présent dans la région conservée du gène *HPV L1* ou *HPV L2* ; et
(ii) ladite deuxième séquence.

5. La méthode selon la revendication 1 ou 4, l'utilisation du système selon la revendication 2 ou 4, ou l'utilisation du kit selon la revendication 3 ou 4, dans laquelle l'ensemble de sondes défini en (iii) comprend en outre :
- une sonde comprenant un fluorophore combiné à une séquence spécifique d'une séquence d'un marqueur du génotype 16 du papillomavirus humain ;
- une sonde comprenant un fluorophore combiné à une séquence spécifique d'une séquence d'un marqueur du génotype 18 du papillomavirus humain.

6. La méthode selon la revendication 1 ou 4, l'utilisation du système selon l'une quelconque des revendications 2, 4 ou 5, ou l'utilisation du kit selon l'une quelconque des revendications 3 à 5, dans laquelle le support solide défini en (b) comprend en outre :
- une sonde comprenant une séquence spécifique d'une séquence d'un marqueur du génotype 16 du papillomavirus humain ;
- une sonde comprenant une séquence spécifique d'une séquence d'un marqueur du génotype 18 du papillomavirus humain.

7. La méthode, l'utilisation du système ou l'utilisation du kit selon la revendication 5 ou 6, dans laquelle ladite PCR multiplexe en temps réel amplifie :
(i) au moins une première séquence, chaque première séquence étant une séquence d'un marqueur d'un génotype du papillomavirus humain choisi dans le groupe constitué de : 16, 18, 31, 33, 35, 39, 45, 51, 52, 56, 58, 59, 66 et 68 ; et
(ii) ladite deuxième séquence.

8. La méthode, l'utilisation du système ou l'utilisation du kit selon les revendications 5 à 7, la méthode, l'utilisation du système ou l'utilisation du kit servant en outre à déterminer si ledit sujet est à risque de développer un cancer du col de l'utérus, de la bouche ou anogénital, dans laquelle lorsque l'un quelconque des génotypes du papillomavirus humain choisis dans le groupe constitué de : 16, 18, 31, 33, 35, 39, 45, 51, 52, 56, 58, 59, 66 et 68, est déterminé, ledit sujet est déterminé comme étant à risque de développer un cancer du col de l'utérus, de la bouche ou anogénital.

9. La méthode selon l'une quelconque des revendications 1, ou 4 à 8, l'utilisation du système selon l'une quelconque des revendications 2, ou 4 à 8, ou l'utilisation du kit selon l'une quelconque des revendications 3 à 8, dans laquelle ledit chauffage est de 60 à 70 °C, ou de 60 à 65 °C.

10. La méthode selon l'une quelconque des revendications 1, ou 4 à 9, l'utilisation du système selon l'une quelconque des revendications 2, ou 4 à 9, ou l'utilisation du kit selon l'une quelconque des revendications 3 à 9, dans laquelle ledit chauffage est de 1 à 20 minutes, ou de 3 à 20 minutes, ou d'au moins 10 minutes.

11. La méthode selon l'une quelconque des revendications 1, ou 4 à 10, l'utilisation du système selon l'une quelconque des revendications 2, ou 4 à 10, ou l'utilisation du kit selon l'une quelconque des revendications 3 à 10, dans laquelle ledit refroidissement consiste à refroidir à 41 °C.

12. La méthode selon l'une quelconque des revendications 1, ou 4 à 11, l'utilisation du système selon l'une quelconque des revendications 2, ou 4 à 11, ou l'utilisation du kit selon l'une quelconque des revendications 3 à 11, dans laquelle l'amorce directe et/ou inverse comprend un marqueur moléculaire.

13. La méthode selon l'une quelconque des revendications 1, 4 à 12, l'utilisation du système selon l'une quelconque des revendications 2, 4 à 12, ou l'utilisation du kit selon l'une quelconque des revendications 3 à 12, dans laquelle ledit échantillon est obtenu au moyen d'un dispositif de prélèvement d'échantillon.

14. La méthode selon l'une quelconque des revendications 1, 4 à 13, l'utilisation du système selon l'une quelconque des revendications 2, 4 à 13, ou l'utilisation du kit selon l'une quelconque des revendications 3 à 13, dans laquelle, avant l'hybridation selon (b), la composition sélectionnée en (a) est chauffée à 95 °C pendant 10 minutes.

15. L'utilisation du système selon l'une quelconque des revendications 2, 4 à 14, ou l'utilisation du kit selon l'une quelconque des revendications 3 à 14, dans laquelle le système et/ou le kit comprennent en outre (aa) des moyens pour extraire l'ADN du papillomavirus humain présent dans un échantillon biologique qui a été obtenu auprès d'un sujet.
